# EUROPEAN PATENT SPECIFICATION

(11) **EP 2 880 083 B1**
(45) Date of publication and mention of the grant of the patent: **21.02.2018**
(21) Application number: 13736587.0
(22) Date of filing: 15.07.2013
(51) Int. Cl.: C08J 3/03, C08J 3/05, C08L 83/04

(54) **AQUEOUS SILICONE DISPERSIONS AND FILMS AND THEIR PREPARATION**
WÄSSRIGE SILIKONDISPERSIONEN UND -FILME UND DEREN HERSTELLUNG
DISPERSIONS AQUEUSES DE SILICONE, FILMS, ET LEUR PRÉPARATION

(30) Priority: 01.08.2012 EP 12305950
(43) Date of publication of application: 10.06.2015
(73) Proprietor: Dow Corning Corporation, Midland, MI 48611 (US); Dow Corning France SAS, 69004 Lyon (FR)
(72) Inventor: CAUVIN, Severine, B-7000 Mons (BE); LE MEUR, Morgane, B-1180 Bruxelles (uccle) (BE); LILES, Donald, Midland, Michigan 48642 (US); THOMAS, Xavier, B-59300 Famars (BE); VINCENT, Anne Marie, B-6210 Les Bons Villers (BE)
(74) Representative: Murgitroyd & Company
(86) International application number: PCT/EP2013/064899
(87) International publication number: WO 2014/019841

(56) References cited:
- EP-A1- 2 267 079
- EP-A2- 0 484 001
- DE-A1-102008 000 585
- US-A1- 2005 119 406

## Description

This invention relates to aqueous silicone dispersions capable of forming a film, to a process for the preparation of such dispersions, and to films formed from such dispersions. The films may be free films or may be films coated on substrates. The invention also relates to a method of treating a mammal by topically applying a composition comprising the aqueous silicone dispersion or a film formed from the dispersion.

US Patent 6,306,411 describes a composition to be applied to the skin and superficial body growths, comprising an aqueous dispersion of particles of film-forming polymer, characterized in that it further comprises an aqueous suspension of particles of at least partially crosslinked solid elastomeric polyorganosiloxane. The elastomeric polyorganosiloxane is obtained by addition reaction and crosslinking in the presence of a catalyst of the platinum type, of at least one polyorganosiloxane containing at least two vinyl groups in position alpha, omega of the silicone chain per molecule, and an organosiloxane containing at least one hydrogen atom linked to a silicon atom per molecule.

US Patent 6,403,704 describes a process for increasing the water-resistance of a cosmetic composition by introducing into the composition particles of an at least partially crosslinked elastomeric polyorganosiloxane suspended in an aqueous phase.

EP-B-1044237 describes an aqueous silicone emulsion useful for preparing anti-adherent coating on paper. Said emulsion comprises polyorganosiloxanes with Si-vinyl units and polyorganosiloxanes with SiH units, cross-linkable by polyaddition in the presence of a platinum catalyst. The emulsion contains a buffer solution for setting and maintaining pH between 5 and 9, an emulsifying agent such as polyvinyl alcohol, and optionally a polyaddition inhibitor. EP-B-587462 and US Patent 5,095,067 describe emulsifying a polyorganosiloxane with Si-vinyl units and a polyorganosiloxane with SiH units together and crosslinking in the presence of a platinum catalyst.

A process according to the present invention for the preparation of an aqueous silicone dispersion comprises mixing in a first step (a) an alkenyl-containing organopolysiloxane having an average per molecule of at least 2 alkenyl groups and (b) an SiH containing siloxane having an average per molecule of at least 2 SiH moieties, then emulsifying the resulting mixture in an aqueous polyvinyl alcohol (PVA) solution to form an aqueous silicone emulsion, wherein a hydrosilylation catalyst is added to the emulsion simultaneously with the aqueous PVA, the alkenyl-containing organopolysiloxane (a) and the SiH containing siloxane (b) being reacted together in the aqueous silicone emulsion and the alkenyl-containing organopolysiloxane (a) and the SiH containing siloxane (b) and their reaction product are stabilised in dispersion form by the PVA dissolved in the aqueous phase, wherein the hydrosilylation reaction takes place within the disperse phase to form a soft elastomer and wherein the aqueous silicone dispersion is a 1-part silicone elastomer emulsion.

By a 'dispersion' we mean a colloidal material having a disperse or discontinuous phase, which may be liquid or solid, dispersed or statistically distributed in a liquid continuous phase. The aqueous silicone dispersion of the invention has a disperse phase of a silicone material, which may be liquid or solid, dispersed in an aqueous liquid continuous phase. An emulsion is a colloidal material having a liquid disperse phase dispersed or statistically distributed in a liquid continuous phase. The alkenyl-containing organopolysiloxane (a) and the SiH containing siloxane (b) are liquids when mixed and when emulsified, but may react within the liquid disperse phase of the emulsion in the presence of the hydrosilylation catalyst to form a solid silicone material. The aqueous dispersion of the invention is capable of forming a film when applied to a substrate.

An aqueous dispersion according to the invention, useful for forming a film, comprises a silicone composition dispersed in an aqueous phase, the silicone composition comprising a product of a reaction of (a) an alkenyl-containing organopolysiloxane having an average per molecule of at least 2 alkenyl groups and (b) an SiH containing siloxane having an average per molecule of at least 2 SiH moieties, the dispersion also comprising a hydrosilylation catalyst and PVA, the silicone composition being stabilised in dispersion form by the PVA dissolved in the aqueous phase.

When the aqueous dispersion disclosed herein is deposited as a film or coated on a substrate and allowed to dry, the disperse phase of reacted silicone composition adheres together as a soft silicone elastomer layer in contact with the substrate. The exposed surface of the film or coating wholly or mainly comprises polyvinyl alcohol. The exposed surface of the film or coating is not tacky. Thus the dispersion is characterizable as being capable of forming a test multilayer film on a flat borosilicate glass substrate, the multilayer film comprising an inner silicone layer sandwiched between, and in operative contact with, the substrate and an outer PVA layer, the inner silicone layer comprising an aggregation of the droplets of the emulsion; and the outer PVA layer being characterizable by a water contact angle (θ) of less than 80 degrees (°). The water contact angle (θ) is measured according to ASTM D7334-08 30 seconds after a test water droplet was deposited thereon.

A multilayer film according to the invention comprises a silicone layer in operative contact with a PVA layer, the silicone layer comprising an aggregation of the droplets of a dispersion as described above; and the PVA layer being characterizable by a water contact angle (θ) of less than 80 degrees (°).

A method of coating a substrate with a multilayer film comprising coating the substrate with an aqueous dispersion as described above and allowing water in the aqueous phase to evaporate from the coating on the substrate.

The dispersion of the invention is effective for use in delivering a pharmaceutically or cosmetically active ingredient by topical application. A pharmaceutical or cosmetic composition according to the invention comprises an admixture of an aqueous dispersion as described above and a pharmaceutically or cosmetically active ingredient, respectively. A pharmaceutically or cosmetically active multilayer film according to the invention comprises a multilayer film as described above, wherein the silicone layer of the multilayer film contains a pharmaceutically or cosmetically active ingredient, respectively.

A method according to the invention of treating a disease or condition in a mammal in need of such treatment comprises topically applying a therapeutically effective amount of the pharmaceutical or cosmetic composition according to the invention as described above to a portion of skin of the mammal. An alternative method of treating a disease or condition in a mammal in need of such treatment comprises topically applying a therapeutically effective amount of the pharmaceutically or cosmetically active multilayer film of the invention to a portion of skin of the mammal.

With or without a pharmaceutically or cosmetically active ingredient, the dispersion of the invention is useful in skin care, for example in masking skin wrinkles. A method according to the invention of masking skin wrinkles in a mammal in need of such treatment comprises topically applying an effective amount of the dispersion composition of the invention to the skin of the mammal. An alternative method of masking skin wrinkles in a mammal in need of such treatment comprises topically applying an effective amount of the multilayer film of the invention to the skin of the mammal.

The alkenyl-containing organopolysiloxane (a) is preferably a substantially linear polydiorganosiloxane but can alternatively be a branched organopolysiloxane. Examples of suitable alkenyl groups include vinyl, hexenyl, allyl, isopropenyl or butenyl groups. The bonding position for the alkenyl groups may be, for example, the terminal position and/or a pendant or side chain position on the molecular chain. Preferably the alkenyl-containing organopolysiloxane is a polydiorganosiloxane containing on average at least two vinyl groups per molecule. The organic groups other than alkenyl groups in the alkenyl-containing organopolysiloxane can for example be alkyl groups having 1 to 12 carbon atoms or aryl groups having 6 to 10 carbon atoms. The organic groups other than alkenyl groups can for example be alkyl groups having 1 to 4 carbon atoms, typically methyl or ethyl groups.

Although the alkenyl-containing organopolysiloxane (a) may contain more than 2, for example 3 up to 6 or more alkenyl groups per molecule, it is often preferred that the alkenyl-containing organopolysiloxane contains only two alkenyl groups per molecule. The alkenyl-containing organopolysiloxane may for example be an alkenyl-terminated polydiorganosiloxane, for example a vinyl-terminated polydimethylsiloxane.

The alkenyl-containing organopolysiloxane (a) can be a branched siloxane, for example of a structure described in EP1070734. The branched siloxane may consist of (i) one or more Q units of the formula (SiO_{4/2}) and ii) from 15 to 995 D units of the formula R*b*₂SiO_{2/2} which units (i) and (ii) may be inter-linked in any appropriate combination, and iii) M units of the formula RaRb ₂SiO_{1/2}, wherein each Ra substituent is selected from the group consisting of an alkyl group having from 1 to 6 carbon atoms and an alkenyl group having up to 6 carbon atoms, and each Rb substituent is selected from the group consisting of an alkyl group having from 1 to 6 carbon atoms, an alkenyl group having 2 to 6 carbon atoms, an aryl group, an alkoxy group, an acrylate group and a methacrylate group. At least two, typically at least three substituents in the branched siloxane are alkenyl groups. Preferably at least three Ra substituents in the branched siloxane are alkenyl groups.

The alkenyl-containing organopolysiloxane (a) preferably has a dynamic viscosity of at least 100 milliPascal-seconds (mPa.s) when tested as described in ASTM D1084-08 Method B (*Standard Test Methods for Viscosity of Adhesives*) or ASTM D4287 - 00(2010) (Standard Test Method for High-Shear Viscosity Using a Cone/Plate Viscometer) at 25°C, and may for example have a dynamic viscosity of from 100 to 100,000,000 mPa.s, particularly 100 to 100,000 mPa.s . It may for example be a vinyl- endcapped polydimethylsiloxane of formula CH₂=CH-Si(CH₃)₂O-[Si(CH₃)₂O]ₙ-Si(CH₃)₂-CH=CH₂, wherein n is an average number of from 100 to 10000, preferably from 100 to 1000.

The alkenyl-containing organopolysiloxane can comprise one or more alkenyl-containing organopolysiloxane as described above. For example it may comprise at least one substantially linear polydiorganosiloxane and at least one branched organopolysiloxane.

Optionally the alkenyl-containing organopolysiloxane (a) comprises an alkenyl-containing organopolysiloxane resin, for example a resin comprising at least one SiO_{4/2} unit and triorganosiloxy units selected from R¹₂R²SiO_{1/2} units and R¹₃SiO_{1/2} units, where R¹ represents a C₁₋₁₀ alkyl group and R² represents an alkenyl group. Each R¹ group can for example be methyl, ethyl, propyl, 2 cyclopentyl or cyclohexyl. Each R² group can for example be vinyl, allyl, isopropenyl, butenyl, hexenyl, or cyclohexenyl, wherein vinyl is preferred. The alkenyl-containing organopolysiloxane resin may for example contain 0.4 to 5.0 mass% alkenyl groups. The alkenyl-containing organopolysiloxane resin can for example comprise 0 to 20wt% of component (a).

The SiH containing siloxane (b) can for example comprise groups selected from RHSiO_{2/2} groups and R2HSiO1_{/2} groups and optionally R₂SiO_{2/2groups} and/or R₃SiO_{1/2} groups, wherein each R denotes an alkyl or aryl group having no more than 8 carbon atoms. The groups R can for example be alkyl groups having 1 to 4 carbon atoms or phenyl groups, typically methyl groups.

The SiH containing siloxane (b) has an average per molecule of at least 2 SiH moieties. Although the invention includes the use of an alkenyl-containing organopolysiloxane (a) containing only 2 alkenyl groups per molecule with a SiH containing siloxane (b) containing only 2 SiH moieties per molecule, it is preferred that either the alkenyl-containing organopolysiloxane (a) contains more than 2 alkenyl groups per molecule or the SiH containing siloxane (b) contains more than 2 SiH moieties per molecule. If the alkenyl-containing organopolysiloxane (a) contains only 2 alkenyl groups per molecule and the SiH containing siloxane (b) contains only 2 SiH moieties per molecule, they will react together in the presence of a hydrosilylation catalyst to undergo chain extension to form a linear polysiloxane of increased molecular weight and increased viscosity, but will in general not undergo crosslinking or form an elastomeric silicone material. If either the alkenyl-containing organopolysiloxane (a) contains more than 2 alkenyl groups per molecule or the SiH containing siloxane (b) contains more than 2 SiH moieties per molecule, they will react together in the presence of a hydrosilylation catalyst to undergo crosslinking, thereby forming an elastomeric silicone material. Preferably the SiH containing siloxane has an average per molecule of more than 2 SiH moieties, for example from 2.5 to 200 SiH moieties, more preferably 3 to 20 SiH moieties.

The SiH containing siloxane (b) can for example be a poly(methylhydrogensiloxane) or a dimethylsiloxane methylhydrogensiloxane copolymer. The SiH containing siloxane can for example comprise 4 to 200 siloxane units and may be an oligomer having 4 to 20 siloxane units. The SiH containing siloxane can for example have a dynamic viscosity at 25ºC of from 1 to 300 mPa.s.

If it is desired that the elastomeric silicone material should contain pendant groups such as hydrocarbon groups having 2 to 30 carbon atoms or polyoxyalkylene groups, the SiH containing siloxane may be modified to contain such groups. For example a poly(methylhydrogensiloxane) or a dimethylsiloxane methylhydrogensiloxane copolymer can be pre-reacted with a hydrocarbon having 2 to 30 carbon atoms and one terminal alkenyl group, for example a 1-alkene, or with a polyoxyalkylene having one terminal alkenyl group, in the presence of a hydrosilylation catalyst. The molar ratio of alkenyl groups to SiH moieties in such pre-reaction must be sufficiently low that the resulting SiH containing siloxane (b) having pendant groups still contains at least 2, preferably more than 2, SiH moieties per molecule.

The molar ratio of SiH moieties of the SiH containing siloxane (b) to alkenyl groups of the alkenyl-containing organopolysiloxane (a) is preferably in the range from 0.5:1 to 1.5:1, more preferably 0.6:1 to 1.2:1. The weight ratio of the SiH containing siloxane (b) to the alkenyl-containing organopolysiloxane (a) may vary widely depending on the reagents used but in general is in the range 1:1000 to 10:1, particularly in the range of 1:200 to 6:1, and is often in the range 1:500 to 1:5.

Catalysts for catalyzing hydrosilylation reactions are known in the art and are commercially available. Such hydrosilylation catalysts can be a metal selected from platinum, rhodium, ruthenium, palladium, osmium, and iridium. Alternatively, the hydrosilylation catalyst may be a compound of such a metal, for example, chloroplatinic acid, chloroplatinic acid hexahydrate, platinum dichloride, and complexes of said compounds with low molecular weight organopolysiloxanes or platinum compounds microencapsulated in a matrix or core/shell type structure. Complexes of platinum with low molecular weight organopolysiloxanes include 1,3-diethenyl-1,1,3,3 -tetramethyldisiloxane complexes with platinum. These complexes may be microencapsulated in a resin matrix. Exemplary hydrosilylation catalysts are described in U.S. Patents 3,159,601; 3,220,972; 3,296,291; 3,419,593; 3,516,946; 3,814,730; 3,989,668; 4,784,879; 5,036,117; and 5,175,325 and EP 0 347 895 B. Microencapsulated hydrosilylation catalysts and methods of preparing them are known in the art, as exemplified in U.S. Patents 4,766,176 and 5,017,654.

The appropriate amount of the catalyst will depend upon the particular catalyst used and the particular alkenyl-containing organopolysiloxane (a) and SiH containing siloxane (b) used. A platinum-containing catalyst may be present in an amount sufficient to provide at least 2 parts per million (ppm) of platinum based on the total weight of the alkenyl-containing organopolysiloxane (a) and the SiH containing siloxane (b) in the composition. Typically, the platinum is present in an amount sufficient to provide 4 to 150 weight ppm of platinum on the same basis. The catalyst may be added as a single species or as a mixture of two or more different species.

The SiH moieties of the SiH containing siloxane (b) and the alkenyl groups of the alkenyl-containing organopolysiloxane (a) react together in the presence of the hydrosilylation catalyst. The product of the reaction is characterised by links between siloxane chains of the formula

=Si - CH2 - CH(Y) - (A)a - Si=

in which the Si atoms shown each form part of different siloxane chains; a = 0 or 1; A if present represents a hydrocarbon linkage usually having 1 to 4 carbon atoms; and Y represents hydrogen or an alkyl group having 1 or 2 carbon atoms. The alkenyl-containing organopolysiloxane (a) and the SiH containing siloxane (b) may be substantially completely reacted so that the reaction product present in the dispersion either contains substantially no unreacted alkenyl groups or contains substantially no unreacted SiH groups (i.e., reacted except for any residual SiH and/or alkenyl that is slow to react for steric hindrance or other reasons), or may be partially reacted.

The PVA can in general be any PVA useful for dispersing the alkenyl-containing organopolysiloxane (a) and the SiH containing siloxane (b) and may be any commercially available polyvinyl alcohol and may for example have a degree of hydrolysis in the range 80% to 99.9%, preferably 85% to 99%. The viscosity of the PVA, measured as the viscosity of a 4 % aqueous solution at 20 °C determined by Hoppler viscometer (DIN 53015), can for example be in the range 3 to 60 mPa.s. Various suitable PVAs are sold by Kuraray America Inc. under the trade mark 'Mowiol', for example Mowiol 18-88, Mowiol 8-88, Mowiol 30-88, Mowiol 30-92 and Mowiol 20-98. Various suitable PVAs are also available from DuPont Inc. under the trade mark 'Elvanol'.

In the first step of the process of the invention, the alkenyl-containing organopolysiloxane (a) and the SiH containing siloxane (b) are mixed together in the absence of any hydrosilylation catalyst. The alkenyl-containing organopolysiloxane (a) and the SiH containing siloxane (b) are both generally liquids. The resulting liquid mixture is then emulsified in an aqueous PVA solution to form an aqueous silicone emulsion in which liquid droplets of the mixture of alkenyl-containing organopolysiloxane (a) and SiH containing siloxane (b) are dispersed in a continuous aqueous phase of PVA solution.

The concentration of the aqueous PVA solution into which the mixture of alkenyl-containing organopolysiloxane (a) and SiH containing siloxane (b) is emulsified can for example be 2 to 40% by weight PVA, preferably 5 to 30%, based on the weight of the aqueous solution. The amount of aqueous PVA solution into which the mixture of alkenyl-containing organopolysiloxane (a) and SiH containing siloxane (b) is emulsified can for example be 2 to 100% by weight based on the weight of the mixture of alkenyl-containing organopolysiloxane (a) and SiH containing siloxane (b). The amount of aqueous PVA solution is preferably 4 to 50% based on the weight of the mixture of alkenyl-containing organopolysiloxane (a) and SiH containing siloxane (b), more preferably 5 to 40%. The amount of PVA thereby mixed in forming the emulsion is preferably in the range from 1.2% to 20% by weight polyvinyl alcohol based on the weight of the mixture of alkenyl-containing organopolysiloxane (a) and SiH containing siloxane (b), more preferably 1.5 to 15% PVA based on the weight of the mixture of alkenyl-containing organopolysiloxane (a) and SiH containing siloxane (b).

At low ratios of aqueous PVA solution to polysiloxane mixture, for example below 15% by weight aqueous PVA solution based on the weight of the mixture of alkenyl-containing organopolysiloxane (a) and SiH containing siloxane (b), a non-Newtonian "thick phase" is formed, which is much more viscous at low shear rate than the silicone polymer alone and often exhibits a yield stress (viscoplastic behaviour). Formation of such a thick phase allows more thorough mixing of the hydrophobic siloxane reagents with the aqueous phase and thus aids in the formation of the emulsion. In the initial stage of the emulsification, the amount of aqueous PVA solution to polysiloxane mixture may be below 15% as defined above for formation of a thick phase. Such a thick phase can for example contain 2 to 10% by weight aqueous PVA solution based on the weight of the mixture of alkenyl-containing organopolysiloxane (a) and SiH containing siloxane (b). Optionally only a part of the PVA is used in the initial stage of the emulsification. The thick phase can be diluted with water or with further PVA solution to form a less viscous emulsion. The concentration of the mixture of alkenyl-containing organopolysiloxane (a) and SiH containing siloxane (b) in the resulting emulsion, diluted if required, can for example be in the range 25 to 90% by weight based on the total weight of the emulsion, typically 40 to 80% based on the total weight of the emulsion.

Emulsification is generally carried out in a high shear mixer, for example a rotor and stator mixer. The particle size of the emulsion can be reduced in a subsequent step if desired, for example in an apparatus applying increased shear such as a homogeniser or microfluidiser, or a sonolator (ultrasonic mixer), producing an emulsion in which the volume median diameter of the droplets is in the range 0.3 to 30µm (micrometres).

The emulsion can be prepared from the aqueous PVA solution into which the mixture of alkenyl-containing organopolysiloxane (a) and SiH containing siloxane (b) is emulsified in the absence of any non-polymeric surfactant. By a 'surfactant' we mean an organic compound that is amphiphilic, that is, it contains within its molecule a portion which is hydrophobic and a portion which is hydrophilic. By 'non-polymeric surfactant', we mean a surfactant of molecular weight below 1600. PVA is not such a surfactant; it is polymeric. PVA may be somewhat amphiphilic due to the presence of residual acetate groups, but it is not necessary to use a PVA which has been modified to be amphiphilic.

The film-forming aqueous silicone dispersion can thus be prepared from the mixture of alkenyl-containing organopolysiloxane (a) and SiH containing siloxane (b), the aqueous polyvinyl alcohol solution and the hydrosilylation catalyst in the absence of any non-polymeric surfactant. We have found that aqueous silicone dispersions prepared by emulsification with PVA solution can be deposited as coherent films having greatly improved mechanical breaking strength compared to films prepared from the same alkenyl-containing organopolysiloxane and SiH containing siloxane reagents using a conventional non-polymeric surfactant, for example an anionic, cationic, non-ionic or amphoteric surfactant. The dispersion can conveniently be deposited by casting but can alternatively be deposited by spraying, spreading or calendaring. In a preferred process according to the invention, no non-polymeric surfactant is used in emulsification or added subsequently to the aqueous silicone dispersion.

A non-polymeric amphiphilic surfactant can however be used in the process of the invention in addition to the aqueous PVA solution. The nonpolymeric surfactant can be a cationic, anionic, nonionic or amphoteric surfactant and can in general be used in an amount such that the weight ratio of PVA to non-polymeric surfactant is at least 2.5:1 on a dry weight basis. If a non-ionic surfactant is present the weight ratio of PVA to nonionic surfactant is preferably at least 5:1, more preferably above 7:1, if it is desired to form a film having sufficient mechanical strength to be handled as a free film. If a cationic or anionic surfactant is present the weight ratio of PVA to cationic or anionic surfactant is preferably at least 2.5:1, more preferably above 5, if it is desired to form a film having sufficient mechanical strength to be handled as a free film. The non-polymeric surfactant is usually present at less than 2% of the total weight of the aqueous silicone dispersion. The non-polymeric surfactant, if used, is usually added to the mixture of alkenyl-containing organopolysiloxane (a) and SiH containing siloxane (b) with the aqueous PVA solution before emulsification but can be added to the aqueous silicone emulsion after emulsification.

Examples of suitable non-ionic surfactants include polyoxyalkylene alkyl ethers such as condensates of ethylene oxide with long chain fatty alcohols or fatty acids such as a C₄₋₁₆ alcohol, particularly polyethylene glycol long chain (12- 14C) alkyl ethers, condensates of ethylene oxide with an amine or an amide, condensation products of ethylene and propylene oxide, esters of glycerol, sucrose, sorbitol, fatty acid alkylol amides, sucrose esters, fluoro-surfactants, fatty amine oxides, polyoxyalkylene sorbitan ethers, polyoxyalkylene alkoxylate esters, and polyoxyalkylene alkylphenol ethers.

Examples of cationic surfactants include quaternary ammonium salts, for example halides such as octyl trimethyl ammonium chloride, dodecyl trimethyl ammonium chloride, hexadecyl trimethyl ammonium chloride, octyl dimethyl benzyl ammonium chloride, decyl dimethyl benzyl ammonium chloride, didodecyl dimethyl ammonium chloride, dioctadecyl dimethyl ammonium chloride, tallow trimethyl ammonium chloride and coco trimethyl ammonium chloride, fatty amines and fatty acid amides and their derivatives, basic pyridinium compounds, quaternary ammonium bases of benzimidazolines and polypropanolpolyethanol amines.

Examples of anionic surfactants include alkyl benzene sulphonic acids and their salts, for example sodium dodecylbenzenesulfonate, and alkyl sulphonic acids and their salts, alkyl sulphates, alkyl ether sulphates, fatty acid ester sulphates, alkyl sulfosuccinates, acyl sarcosinates, alkyl carboxylates, fatty acids, and phosphate esters in acid or salt form.

The alkenyl-containing organopolysiloxane (a) and the SiH containing siloxane (b) can if desired be mixed before emulsification with an excipient oil. By an 'oil' we mean a liquid which is immiscible with water. The excipient oil should be miscible with the mixture of alkenyl-containing organopolysiloxane (a) and SiH containing siloxane (b). The excipient oil generally contains no functional groups which are reactive with the alkenyl groups of alkenyl-containing organopolysiloxane (a) or the SiH groups of SiH containing siloxane (b) under the conditions of emulsification. The oil may for example be a softener or plasticiser for the elastomeric silicone that is to be formed, or may be an oil having benefit in skin care, for example as an emollient. The oil may be an excipient for a pharmaceutically or cosmetically active material that is to be incorporated into the silicone dispersion; the oil may be a mixture of such an excipient and a pharmaceutically or cosmetically active material. The amount of such an oil mixed before emulsification can be up to 50% by weight based on the weight of the alkenyl-containing organopolysiloxane (a) and the SiH containing siloxane (b), for example 1 to 40% and preferably 2 to 20% by weight based on the weight of the alkenyl-containing organopolysiloxane (a) and the SiH containing siloxane (b).

The hydrosilylation catalyst is preferably added to the emulsion simultaneously with the aqueous PVA, for example by mixing the catalyst into the aqueous PVA solution before emulsification, although the catalyst can be added to the emulsion after emulsification if desired. When the catalyst contacts the mixture of alkenyl-containing organopolysiloxane (a) and SiH containing siloxane (b), reaction of the alkenyl groups of (a) with the SiH moieties of (b) is initiated. Polymerisation of the alkenyl-containing organopolysiloxane (a) and SiH containing siloxane (b) thus takes place within the liquid disperse phase of the emulsion (emulsion polymerisation). Polymerisation takes place at ambient temperature in the presence of the hydrosilylation catalyst. Ambient temperature polymerisation may be preferred for convenience, although any temperature in the range 0 to 100ºC can be used. An elevated temperature, for example in the range 50 to 100ºC, may be preferred to give more rapid polymerisation.

It may be preferred that the pH of the dispersion is below pH6, more particularly below pH5. This avoids the possibility of hydrolysis of PVA in the presence of a platinum catalyst so that the dispersion remains stable on storage and retains its properties such as the mechanical properties and contact angle of a film deposited from the dispersion. A buffering agent such as citric acid with sodium hydroxide can be added to the emulsion to control the pH to the desired value.

A pharmaceutically or cosmetically active material, optionally in admixture with an excipient, can be added to the emulsion at any time after emulsification. The amount of pharmaceutically or cosmetically active material, including any excipient, can be up to 50% by weight based on the weight of the alkenyl-containing organopolysiloxane (a) and the SiH containing siloxane (b), for example 1 to 40% and preferably 2 to 20% by weight based on the weight of the alkenyl-containing organopolysiloxane (a) and the SiH containing siloxane (b).

A filler can be added to the emulsion at any time after emulsification. A filler can for example be a reinforcing filler such as hydrophilic silica or can be a cosmetic filler, for example a silicone crosspolymer powder with silica treated coating or a silicone elastomer powder. The filler can be added as a particulate solid or can be added as a suspension, for example a nonionic aqueous suspension of a silicone elastomer powder.

The composition of the aqueous silicone dispersion of the invention thus comprises 5 to 90% by weight of the alkenyl-containing organopolysiloxane (a); 0.025 to 75% by weight of the SiH containing siloxane (b); 0.0002 to 0.02% by weight of the hydrosilylation catalyst; 0.3 to 18% by weight PVA; 8 to 94% by weight water; and optionally 0 to 50% by weight water immiscible oil which is miscible with but not reactive with the alkenyl-containing organopolysiloxane (a) or the SiH containing siloxane (b); and/or 0 to 50% by weight pharmaceutically or cosmetically active material including any excipient therefore; and/or 0 to 0.5% by weight of a nonionic surfactant; and/or 0 to 1.5% by weight of a cationic surfactant, all based on the total weight of the aqueous silicone dispersion.

A "pharmaceutically active" material means any compound or mixtures of compounds that provide a pharmaceutical or medical benefit. Thus, pharmaceutically active materials include materials consider as an active ingredient or active drug ingredient as generally used and defined by the United States Department of Health & Human Services Food and Drug Administration, contained in Title 21, Chapter I, of the Code of Federal Regulations, Parts 200-299 and Parts 300-499. A 'cosmetically active' material means any compound or mixtures of compounds that are additives in personal care formulations added for the purpose of treating hair or skin to provide a cosmetic and/or aesthetic benefit.

The pharmaceutically active material can include any component that is intended to furnish pharmacological activity or other direct effect in the diagnosis, cure, mitigation, treatment, or prevention of disease, or to affect the structure or any function of the body of a human or other animals. The pharmaceutically active material can include those components that may undergo chemical change in the manufacture of drug products and be present in drug products in a modified form intended to furnish the specified activity or effect.

Some representative examples of pharmaceutically active materials include: drugs, vitamins, minerals; hormones; topical antimicrobial agents such as antibiotic active ingredients, antifungal active ingredients for the treatment of athlete's foot, jock itch, or ringworm, and acne active ingredients; astringent active ingredients; deodorant active ingredients; wart remover active ingredients; corn and callus remover active ingredients; pediculicide active ingredients for the treatment of head, pubic (crab), and body lice; active ingredients for the control of dandruff, seborrheic dermatitis, or psoriasis; and sunburn prevention and treatment agents.

Examples of cosmetically active materials include emollients, waxes, moisturizers, sebum absorbants or sebum control agents, vegetable or botanical extracts, pigments, colorants, conditioning agents, UV absorbers and sunscreen agents, proteins and amino-acids and their derivatives, fragrances, antiperspirants, colour care additives, pearlising agents, antioxidants, skin bleaching agents and skin protectants.

Examples of vitamins include a variety of different organic compounds such as alcohols, acids, sterols, and quinones. They may be classified into two solubility groups: lipid-soluble vitamins and water-soluble vitamins. Lipid-soluble vitamins that have utility in personal care formulations include retinol (vitamin A), ergocalciferol (vitamin D2), cholecalciferol (vitamin D3), phytonadione (vitamin K1), and tocopherol (vitamin E). Water-soluble vitamins that have utility in personal care formulations include ascorbic acid (vitamin C), thiamin (vitamin B1), niacin (nicotinic acid), niacinamide (vitamin B3), riboflavin (vitamin B2), pantothenic acid (vitamin B5), biotin, folic acid, pyridoxine (vitamin B6), and cyanocobalamin (vitamin B12). Additional examples of vitamins include derivatives of vitamins such as retinyl palmitate (vitamin A palmitate), retinyl acetate (vitamin A acetate), retinyl linoleate (vitamin A linoleate), and retinyl propionate (vitamin A propionate), tocopheryl acetate (vitamin E acetate), tocopheryl linoleate (vitamin E linoleate), tocopheryl succinate (vitamin E succinate), tocophereth-5, tocophereth-10, tocophereth-12, tocophereth-18, tocophereth-50 (ethoxylated vitamin E derivatives), PPG-2 tocophereth-5, PPG-5 tocophereth-2, PPG-10 tocophereth-30, PPG-20 tocophereth-50, PPG-30 tocophereth-70, PPG-70 tocophereth-100 (propoxylated and ethoxylated vitamin E derivatives), sodium tocopheryl phosphate, ascorbyl palmitate, ascorbyl dipalmitate, ascorbyl glucoside, ascorbyl tetraisopalmitate, tetrahexadecyl ascorbate, ascorbyl tocopheryl maleate, potassium ascorbyl tocopheryl phosphate or tocopheryl nicotinate.

The pharmaceutically active material used in processes according to the invention can be an active drug ingredient. Representative examples of some suitable active drug ingredients which can be used are hydrocortisone, ketoprofen, timolol, pilocarpine, adriamycin, mitomycin C, morphine, hydromorphone, diltiazem, theophylline, doxorubicin, daunorubicin, heparin, penicillin G, carbenicillin, cephalothin, cefoxitin, cefotaxime, 5-fluorouracil, cytarabine, 6-azauridine, 6-thioguanine, vinblastine, vincristine, bleomycin sulfate, aurothioglucose, suramin, mebendazole, clonidine, scopolamine, propranolol, phenylpropanolamine hydrochloride, ouabain, atropine, haloperidol, isosorbide, nitroglycerin, ibuprofen, ubiquinones, indomethacin, prostaglandins, naproxen, salbutamol, guanabenz, labetalol, pheniramine, metrifonate, and steroids. Active drug ingredients for purposes of the present invention also include antiacne agents such as benzoyl peroxide and tretinoin; antibacterial agents such as chlorohexadiene gluconate; antifungal agents such as miconazole nitrate; anti-inflammatory agents; corticosteroidal drugs; non-steroidal anti-inflammatory agents such as diclofenac; antipsoriasis agents such as clobetasol propionate; anesthetic agents such as lidocaine; antipruritic agents; and antidermatitis agents.

The pharmaceutically active material can be a protein, such as an enzyme. Enzymes include, but are not limited to, commercially available types, improved types, recombinant types, wild types, variants not found in nature, and mixtures thereof. For example, suitable enzymes include hydrolases, cutinases, oxidases, transferases, reductases, hemicellulases, esterases, isomerases, pectinases, lactases, peroxidases, laccases, catalases, and mixtures thereof. Hydrolases include, but are not limited to, proteases (bacterial, fungal, acid, neutral or alkaline), amylases (alpha or beta), lipases, mannanases, cellulases, collagenases, lisozymes, superoxide dismutase, catalase, and mixtures thereof. Said proteases include, but are not limited to, trypsin, chymotrypsin, pepsin, pancreatin and other mammalian enzymes; papain, bromelain and other botanical enzymes; subtilisin, epidermin, nisin, naringinase(L-rhammnosidase) urokinase and other bacterial enzymes. Said lipases include, but are not limited to, triacyl-glycerol lipases, monoacyl-glycerol lipases, lipoprotein lipases, e.g. steapsin, erepsin, pepsin, other mammalian, botanical, bacterial lipases and purified ones. Natural papain is preferred as said enzyme. Further, stimulating hormones, e.g. insulin, can be used together with these enzymes to boost the effectiveness of them.

The pharmaceutically or cosmetically active material may be a sunscreen agent. The sunscreen agent can be selected from any sunscreen agent known in the art to protect skin from the harmful effects of exposure to sunlight. The sunscreen compound is typically chosen from an organic compound, an inorganic compound, or mixtures thereof, that absorbs ultraviolet (UV) light. UV absorbers and sunscreen agents include those which absorb ultraviolet light between about 290-320 nanometers (the UV-B region) and those which absorb ultraviolet light in the range of 320-400 nanometers (the UV-A region).

Some examples of sunscreen agents are aminobenzoic acid, cinoxate, diethanolamine methoxycinnamate, digalloyl trioleate, dioxybenzone, ethyl 4-[bis(Hydroxypropyl)] aminobenzoate, glyceryl aminobenzoate, homosalate, lawsone with dihydroxyacetone, menthyl anthranilate, octocrylene, ethyl hexyl methoxycinnamate, octyl salicylate, oxybenzone, padimate O, phenylbenzimidazole sulfonic acid, red petrolatum, sulisobenzone, titanium dioxide, and trolamine salicylate.

Further examples of UV absorbers are acetaminosalol, allatoin PABA, benzalphthalide, benzophenone, benzophenone 1-12, 3-benzylidene camphor, benzylidenecamphor hydrolyzed collagen sulfonamide, benzylidene camphor sulfonic acid, benzyl salicylate, bornelone, bumetriozole, butyl Methoxydibenzoylmethane, butyl PABA, ceria/silica, ceria/silica talc, cinoxate, DEA-methoxycinnamate, dibenzoxazol naphthalene, di-t-butyl hydroxybenzylidene camphor, digalloyl trioleate, diisopropyl methyl cinnamate, dimethyl PABA ethyl cetearyldimonium tosylate, dioctyl butamido triazone, diphenyl carbomethoxy acetoxy naphthopyran, disodium bisethylphenyl tiamminotriazine stilbenedisulfonate, disodium distyrylbiphenyl triaminotriazine stilbenedisulfonate, disodium distyrylbiphenyl disulfonate, drometrizole, drometrizole trisiloxane, ethyl dihydroxypropyl PABA, ethyl diisopropylcinnamate, ethyl methoxycinnamate, ethyl PABA, ethyl urocanate, etrocrylene ferulic acid, glyceryl octanoate dimethoxycinnamate, glyceryl PABA, glycol salicylate, homosalate, isoamyl p-methoxycinnamate, isopropylbenzyl salicylate, isopropyl dibenzolylmethane, isopropyl methoxycinnamate, menthyl anthranilate, menthyl salicylate, 4-methylbenzylidene, camphor, octocrylene, octrizole, octyl dimethyl PABA, ethyl hexyl methoxycinnamate, octyl salicylate, octyl triazone, PABA, PEG-25 PABA, pentyl dimethyl PABA, phenylbenzimidazole sulfonic acid, polyacrylamidomethyl benzylidene camphor, potassium methoxycinnamate, potassium phenylbenzimidazole sulfonate, red petrolatum, sodium phenylbenzimidazole sulfonate, sodium urocanate, TEA-phenylbenzimidazole sulfonate, TEA-salicylate, terephthalylidene dicamphor sulfonic acid, titanium dioxide, triPABA panthenol, urocanic acid, and VA/crotonates/methacryloxybenzophenone-1 copolymer.

The cosmetically active material may be a fragrance or perfume. The perfume can be any perfume or fragrance active ingredient commonly used in the perfume industry. These compositions typically belong to a variety of chemical classes, as varied as alcohols, aldehydes, ketones, esters, ethers, acetates, nitrites, terpenic hydrocarbons, heterocyclic nitrogen or sulfur containing compounds, as well as essential oils of natural or synthetic origin. Many of these perfume ingredients are described in detail in standard textbook references such as Perfume and Flavour Chemicals, 1969, S. Arciander, Montclair, N.J. Fragrances may be exemplified by, but not limited to, perfume ketones and perfume aldehydes. Illustrative of the perfume ketones are buccoxime; iso jasmone; methyl beta naphthyl ketone; musk indanone; tonalid/musk plus; Alpha-Damascone, Beta-Damascone, Delta-Damascone, Iso-Damascone, Damascenone, Damarose, Methyl-Dihydrojasmonate, Menthone, Carvone, Camphor, Fenchone, Alpha-ionone, Beta-lonone, Gamma-Methyl so-called lonone, Fleuramone, Dihydrojasmone, Cis-Jasmone, Iso-E-Super, Methyl-Cedrenyl-ketone or Methyl-Cedrylone, Acetophenone, Methyl-Acetophenone, Para-MethoxyAcetophenone, Methyl-Beta-Naphtyl-Ketone, Benzyl-Acetone, Benzophenone, Para-Hydroxy-Phenyl-Butanone, Celery Ketone or Livescone, 6-Isopropyldecahydro-2-naphtone, Dimethyl-Octenone, Freskomenthe, 4-(1-Ethoxyvinyl)-3,3,5,5-tetramethyl-Cyclohexanone, Methyl-Heptenone, 2-(2-(4-Methyl-3-cyclohexen-1-yl)propyl)-cyclopentanone, 1-(p-Menthen-6(2)-yl)-1-propanone, 4-(4-Hydroxy-3-methoxyphenyl)-2-butanone, 2-Acetyl-3,3-Dimethyl-Norbomane, 6,7-Dihydro-1,1,2,3,3-Pentamethyl-4(5H)-Indanone, 4-Damascol, Dulcinyl or Cassione, Gelsone, Hexalon, Isocyclemone E, Methyl Cyclocitrone, Methyl-Lavender-Ketone, Orivon, Para-tertiary-Butyl-Cyclohexanone, Verdone, Delphone, Muscone, Neobutenone, Plicatone, Veloutone, 2,4,4,7-Tetramethyl-oct-6-en-3-one, and Tetrameran. Examples of perfume aldehydes are adoxal; anisic aldehyde; cymal; ethyl vanillin; florhydral; helional; heliotropin; hydroxycitronellal; koavone; lauric aldehyde; lyral; methyl nonyl acetaldehyde; P.T. bucinal; phenyl acetaldehyde; undecylenic aldehyde; vanillin; 2,6,10-trimethyl-9-undecenal, 3-dodecen-1-al, alpha-n-amyl cinnamic aldehyde, 4-methoxybenzaldehyde, benzaldehyde, 3-(4-tert butylphenyl)-propanal, 2-methyl-3-(para-methoxyphenyl propanal, 2-methyl-4-(2,6,6-trimethyl-2(1)-cyclohexen-1-yl) butanal, 3-phenyl-2-propenal, cis-/trans-3,7-dimethyl-2,6-octadien-1-al, 3,7-dimethyl-6-octen-1-al, [(3,7-dimethyl-6-octenyl)oxy] acetaldehyde, 4-isopropylbenzyaldehyde, 1,2,3,4,5,6,7,8-octahydro-8,8-dimethyl-2-naphthaldehyde, 2,4-dimethyl-3-cyclohexen-1-carboxaldehyde, 2-methyl-3-(isopropylphenyl)propanal, 1-decanal; decyl aldehyde, 2,6-dimethyl-5-heptenal, 4-(tricyclo[5.2.1.0(2,6)]-decylidene-8)-butanal, octahydro-4,7-methano-1H-indenecarboxaldehyde, 3-ethoxy-4-hydroxy benzaldehyde, para-ethyl-alpha, alpha-dimethyl hydrocinnamaldehyde, alpha-methyl-3,4-(methylenedioxy)-hydrocinnamaldehyde, 3,4-methylenedioxybenzaldehyde, alpha-n-hexyl cinnamic aldehyde, m-cymene-7-carboxaldehyde, alpha-methyl phenyl acetaldehyde, 7-hydroxy-3,7-dimethyl octanal, Undecenal, 2,4,6-trimethyl-3-cyclohexene-1-carboxaldehyde, 4-(3) (4-methyl-3-pentenyl)-3-cyclohexen-carboxaldehyde, 1-dodecanal, 2,4-dimethyl cyclohexene-3-carboxaldehyde, 4-(4-hydroxy-4-methyl pentyl)-3-cylohexene-1-carboxaldehyde, 7-methoxy-3,7-dimethyloctan-1-al, 2-methyl undecanal, 2-methyl decanal, 1-nonanal, 1-octanal, 2,6,10-trimethyl-5,9-undecadienal, 2-methyl-3-(4-tertbutyl)propanal, dihydrocinnamic aldehyde, 1-methyl-4-(4-methyl-3-pentenyl)-3-cyclohexene-1-carbox aldehyde, 5 or 6 methoxyl 0 hexahydro-4,7-methanoindan-1 or 2-carboxaldehyde, 3,7-dimethyloctan-1-al, 1-undecanal, 10-undecen-1-al, 4-hydroxy-3-methoxy benzaldehyde, 1-methyl-3-(4-methylpentyl)-3-cyclhexenecarboxaldehyde, 7-hydroxy-3,7-dimethyl-octanal, trans-4-decenal, 2,6-nonadienal, paratolylacetaldehyde; 4-methylphenylacetaldehyde, 2-methyl-4-(2,6,6-trimethyl-1-cyclohexen-1-yl)-2-butena 1, ortho-methoxycinnamic aldehyde, 3,5,6-trimethyl-3-cyclohexene carboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanoindan-1-carboxaldehyde, 2-methyl octanal, alpha-methyl-4-(1-methyl ethyl) benzene acetaldehyde, 6,6-dimethyl-2-norpinene-2-propionaldehyde, para methyl phenoxy acetaldehyde, 2-methyl-3-phenyl-2-propen-1-al, 3,5,5-trimethyl hexanal, Hexahydro-8,8-dimethyl-2-naphthaldehyde, 3-propyl-bicyclo[2.2.1]-hept-5-ene-2-carbaldehyde, 9-decenal, 3-methyl-5-phenyl-1-pentanal, methylnonyl acetaldehyde, hexanal, trans-2-hexenal, 1-p-menthene-q-carboxaldehyde, hexyl cinnamic aldehyde and mixtures thereof. Further examples of fragrances or perfumes include methyl- 2-n-hexyl-3-oxo-cyclopentane carboxylate; gamma-dodecalactone; methylphenylcarbinyl acetate; 4-acetyl-6-tert-butyl-1 ,1 -dimethyl indane; patchouli; olibanum resinoid; labdanum; vetivert; copaiba balsam; fir balsam; methyl anthranilate; geraniol; geranyl acetate; linalool; citronellol; terpinyl acetate; benzyl salicylate; phenoxyethyl isobutyrate; cedryl acetal; aubepine; and ethylene brassylate.

Examples of vegetable or botanical extracts are derived from plants (herbs, roots, flowers, fruits, or seeds) in oil or water soluble form, such as coconut, green tea, white tea, black tea, horsetail, sunflower, wheat germ, olive, grape, pomegranate, apricot, carrot, tomato, tobacco, bean, potato, adzuki bean, catechu, orange, cucumber, avocado, watermelon, banana, lemon, palm, dill, horseradish, oats, neem, beet, broccoli, pumpkin, soybean, barley, walnut, flax, ginseng, poppy, avocado, pea or sesame extract.

Examples of emollients include volatile or non-volatile silicone oils; silicone resins such as polypropylsilsesquioxane and phenyl trimethicone; silicone elastomers such as dimethicone crosspolymer; alkylmethylsiloxanes such as C30-45 Alkyl Methicone; volatile or non-volatile hydrocarbon compounds, such as squalene, paraffin oils, petrolatum oils and naphthalene oils; hydrogenated or partially hydrogenated polyisobutene; isoeicosane; squalane; isoparaffin; isododecane; isodecane or isohexadecane; branched C₈-C₁₆ esters; isohexyl neopentanoate; ester oils such as isononyl isononanoate, cetostearyl octanoate, isopropyl myristate, palmitate derivatives, stearate derivatives, isostearyl isostearate and the heptanoates, octanoates, decanoates or ricinoleates of alcohols or of polyalcohols, or mixtures thereof; oils of plant origin, such as wheatgerm, sunflower, grapeseed, castor, shea, avocado, olive, soybean, sweet almond, palm, rapeseed, cotton seed, hazelnut, macadamia, jojoba, blackcurrant, or evening primrose oil; triglycerides of caprylic/capric acids; or higher fatty acids, such as oleic acid, linoleic acid or linolenic acid.

Examples of waxes include beeswax, lanolin wax, rice wax, carnauba wax, candelilla wax, and hydrocarbon waxes such as microcrystalline waxes, paraffins, ozokerite, polyethylene waxes.

Examples of moisturizers include lower molecular weight aliphatic diols such as propylene glycol and butylene glycol; polyols such as glycerine and sorbitol; and polyoxyethylene polymers such as polyethylene glycol 200; and hyaluronic acid and its derivatives.

Examples of sebum absorbants or sebum control agents include silica silylate, silica dimethyl silylate, dimethicone/vinyl dimethicone crosspolymer, polymethyl methacrylate, cross-linked methylmethacrylate and aluminum starch octenylsuccinate.

Examples of conditioning agents include silicone conditioning agents such as silicone oils, silicone gums and mixtures thereof; organomodified silicone oils, such as amodimethicone, aminopropyl phenyl trimethicone, phenyl trimethicone, trimethyl pentaphenyl trisiloxane, silicone quaternium-16/glycidoxy dimethicone crosspolymer, silicone quaternium-16 and mixtures thereof. Further examples of conditioning agents are cationic conditioning agents including guar derivatives; quaternary nitrogen derivatives of cellulose ethers; homopolymers of dimethyldiallyl ammonium chloride; copolymers of acrylamide and dimethyldiallyl ammonium chloride; homopolymers or copolymers derived from acrylic acid or methacrylic acid which contain cationic nitrogen functional groups attached to the polymer by ester or amide linkages; polycondensation products of N,N'-bis-(2,3-epoxypropyl)-piperazine or piperazine-bis-acrylamide and piperazine; and copolymers of vinylpyrrolidone and acrylic acid esters with quaternary nitrogen functionality.

Proteins or amino-acids and their derivatives suitable for use as cosmetically active materials include proteins extracted from wheat, soy, rice, corn, keratin, elastin or silk and amino-acids derived therefrom. The protein may be in the hydrolyzed form. The protein may be quaternized.

Some examples of antioxidants suitable for use as cosmetically active materials are acetyl cysteine, arbutin, ascorbic acid, ascorbic acid polypeptide, ascorbyl dipalmitate, ascorbyl methylsilanol pectinate, ascorbyl palmitate, ascorbyl stearate, BHA, p-hydroxyanisole, BHT, t-butyl hydroquinone, caffeic acid, Camellia sinensis Oil, chitosan ascorbate, chitosan glycolate, chitosan salicylate, chlorogenic acids, cysteine, cysteine HCI, decyl mercaptomethylimidazole, erythorbic acid, diamylhydroquinone, di-t-butylhydroquinone, dicetyl thiodipropionate, dicyclopentadiene/t-butylcresol copolymer, digalloyl trioleate, dilauryl thiodipropionate, dimyristyl thiodipropionate, dioleyl tocopheryl methylsilanol, isoquercitrin, diosmine, disodium ascorbyl sulfate, disodium rutinyl disulfate, distearyl thiodipropionate, ditridecyl thiodipropionate, dodecyl gallate, ethyl ferulate, ferulic acid, hydroquinone, hydroxylamine HCI, hydroxylamine sulfate, isooctyl thioglycolate, kojic acid, madecassicoside, magnesium ascorbate, magnesium ascorbyl phosphate, melatonin, methoxy-PEG-7 rutinyl succinate, methylene di-t-butylcresol, methylsilanol ascorbate, nordihydroguaiaretic acid, octyl gallate, phenylthioglycolic acid, phloroglucinol, potassium ascorbyl tocopheryl phosphate, thiodiglycolamide, potassium sulfite, propyl gallate, rosmarinic acid, rutin, sodium ascorbate, sodium ascorbyl/cholesteryl phosphate, sodium bisulfite, sodium erythorbate, sodium metabisulfide, sodium sulfite, sodium thioglycolate, sorbityl furfural, tea tree (Melaleuca aftemifolia) oil, tocopheryl acetate, tetrahexyldecyl ascorbate, tetrahydrodiferuloylmethane, tocopheryl linoleate/oleate, thiodiglycol, tocopheryl succinate, thiodiglycolic acid, thioglycolic acid, thiolactic acid, thiosalicylic acid, thiotaurine, retinol, tocophereth-5, tocophereth-10, tocophereth-12, tocophereth-18, tocophereth-50, tocopherol, tocophersolan, tocopheryl linoleate, tocopheryl nicotinate, tocoquinone, o-tolyl biguanide, tris(nonylphenyl) phosphite, ubiquinone, and zinc dibutyldithiocarbamate.

An example of a skin bleaching agent is hydroquinone. Some examples of skin protectants are allantoin, aluminium acetate, aluminium hydroxide, aluminium sulfate, calamine, cocoa butter, cod liver oil, colloidal oatmeal, dimethicone, glycerin, kaolin, lanolin, mineral oil, petrolatum, shark liver oil, sodium bicarbonate, talc, witch hazel, zinc acetate, zinc carbonate, and zinc oxide.

An excipient used with such a pharmaceutically or cosmetically active material is generally selected from organic liquids (oils and solvents), silicones and mixtures of these. Many of the liquid organic and silicone materials listed above as emollients are also suitable as excipients for pharmaceutically or cosmetically active materials. Organic liquids suitable as excipients are exemplified by, but not limited to, aromatic hydrocarbons, aliphatic hydrocarbons, alcohols, aldehydes, ketones, amines, esters, ethers, glycols, glycol ethers, alkyl halides and aromatic halides. Hydrocarbons include isododecane, isohexadecane, Isopar L (C11 -C13), Isopar H (C11 -C12) and other mineral oils, petrolatum and hydrogenated polydecene. Ethers and esters include isodecyl neopentanoate, neopentylglycol heptanoate, glycol distearate, dicaprylyl carbonate, dicaprylyl ether, diethylhexyl carbonate, propylene glycol n butyl ether, ethyl-3 ethoxypropionate, propylene glycol methyl ether acetate, tridecyl neopentanoate, propylene glycol methylether acetate (PGMEA), propylene glycol methylether (PGME), octyldodecyl neopentanoate, diisobutyl adipate, diisopropyl adipate, isopropyl myristate, isopropyl palmitate, isopropyl isostearate, propylene glycol dicaprylate/dicaprate, caprylic/capric triglyceride and octyl palmitate. Examples of alcohols include glycerol, ethanol, pentylene glycol and propylene glycol. Additional organic carrier fluids suitable as an ingredient of the excipient include fats, oils, fatty acids, and fatty alcohols.

The excipient may be a low viscosity organopolysiloxane having a viscosity at 25° C in the range of 1 to 1,000 mPa.s such as decamethylcyclopentasiloxane, dodecamethylcyclohexasiloxane, octamethyltrisiloxane, decamethyltetrasiloxane, dodecamethylpentasiloxane, tetradecamethylhexasiloxane, hexadeamethylheptasiloxane, heptamethyl-3-{(trimethylsilyl)oxy)}trisiloxane, hexamethyl-3,3,bis{(trimethlylsilyl)oxy}trisiloxane pentamethyl{(trimethylsilyl)oxy}cyclotrisiloxane as well as polydimethylsiloxanes, polydiethylsiloxanes, polymethylethylsiloxanes, polymethylphenylsiloxanes, or polydiphenylsiloxanes.

A film can be formed according to the invention by coating the aqueous silicone dispersion of the invention on a substrate and allowing water in the aqueous phase to evaporate from the coating on the substrate to form a film. The substrate can be a casting surface from which the film is removed as a freestanding film or can be a substrate which requires coating by a film according to the invention. The film produced is a multilayer film comprising a silicone layer in operative contact with a PVA layer. The silicone layer comprises an aggregation of the droplets of the aqueous silicone dispersion. The PVA layer is characterizable by a water contact angle (θ) of less than 80 degrees (°) when θ is measured according to ASTM D7334-08 30 seconds after a test water droplet was deposited thereon.

Thus the aqueous silicone dispersion of the invention is characterizable as being capable of forming a test multilayer film on a flat borosilicate glass substrate, the multilayer film comprising an inner silicone layer sandwiched between, and in operative contact with, the substrate and an outer PVA layer, the inner silicone layer comprising an aggregation of the droplets of the dispersion; and the outer PVA layer being characterizable by a water contact angle (θ) of less than 80 degrees (°) when θ is measured according to ASTM D7334-08 30 seconds after a test water droplet was deposited thereon.

When the aqueous silicone dispersion of the invention is deposited on a substrate and water is allowed to evaporate, the coating film formed on the substrate comprises a silicone layer in contact with the substrate so as to sandwich the silicone layer between the substrate and a PVA layer. In a coated substrate according to the invention, the substrate is coated with a multilayer film comprising a silicone layer in operative contact with a PVA layer, wherein the silicone layer of the multilayer film is in contact with the substrate so as to sandwich the silicone layer between the substrate and the PVA layer. A dry tack-free film may for example be achieved in 5 to 10 minutes after deposition of the aqueous silicone dispersion of the invention on a substrate.

We have found that the film or coating of the invention can be either shiny or matte, depending on the particle size of the dispersion, the grade of PVA used and the proportion of PVA to silicone in the film. A higher proportion of PVA tends to form smaller dispersed particles and a more shiny film or coating; a lower proportion of PVA tends to form larger dispersed particles and a more matte film or coating. The proportion of PVA can be controlled to determine whether the film or coating is shiny or matte. This may be advantageous in cosmetic applications. The grade and the particle size of the PVA dispersion used also influence the aspect of the film or coating and can be chosen to give the desired film appearance. A more fully hydrolysed PVA tends to produce a matte film. A higher molecular weight PVA tends to produce a shiny film.

The silicone layer of the film according to the invention is preferably crosslinked to an elastomeric silicone material, by reaction of the alkenyl-containing organopolysiloxane (a) and the SiH containing siloxane (b) in the disperse phase of the dispersion in the presence of the hydrosilylation catalyst. The silicone layer preferably comprises 60 to 99% by weight of the multilayer film on a dry weight basis.

The aqueous silicone dispersion of the invention is thus a 1-part silicone elastomer emulsion that forms a film upon drying after deposition. A hydrosilylation reaction takes place within the disperse phase to form a soft elastomer. Upon drying, a film is formed without the need of further reaction contrarily to most hydrosilylation film forming technology which requires a two parts system which reacts after deposition. This is partly because the PVA has film forming properties in addition to acting as an emulsion stabiliser, and partly because the elastomeric silicone disperse phase droplets are soft enough to adhere together. A further benefit of the aqueous silicone dispersion of the invention is that only a low amount of platinum catalyst is required, as the hydrosilylation reaction takes place within the disperse phase and a fast cure is not required for film formation.

We have found that the multilayer films of the invention have substantially increased mechanical resistance compared to films prepared from emulsions and dispersions of similar organopolysiloxane and siloxane reagents stabilised by an amphiphilic surfactant in place of the PVA.

The aqueous silicone dispersion of the invention can be used in pharmaceutical and cosmetic treatment in a variety of ways for delivering a pharmaceutically or cosmetically active ingredient to a patient by topical application. A pharmaceutical or cosmetic composition comprising an admixture of the aqueous silicone dispersion and a pharmaceutically or cosmetically active ingredient, respectively, can be used to treat a disease or condition in a mammal in need of such treatment by topically applying a therapeutically effective amount of the composition to a portion of skin of the mammal. The composition forms a film on the skin of the mammal from which the pharmaceutically or cosmetically active ingredient is absorbed onto and through the skin. Alternatively the pharmaceutical or cosmetic composition comprising an admixture of the aqueous silicone dispersion and a pharmaceutically or cosmetically active ingredient can be deposited as a film, and the resulting pharmaceutically or cosmetically active multilayer film can be used to treat a disease or condition in a mammal in need of such treatment by topically applying a therapeutically effective amount of the film to a portion of skin of the mammal. The pharmaceutically or cosmetically active ingredient is absorbed from the film onto and through the skin of the mammal.

Personal care compositions in which the dispersion of the invention can be used to deliver a cosmetically active ingredient include skin care compositions, hair care compositions and nail care compositions. Skin care compositions include shower gels, soaps, hydrogels, creams, lotions, balms, foundations, lipsticks, eyeliners and blushes, primer, concealer, correctors and pencils. The benefits of using the silicone aqueous dispersion of the invention in skin care compositions may include skin hydration, protection, long lasting, skin adhesion, SPF (sun protection factor) boosting, wash off resistance, tensing, and/or tightening. Hair care compositions include shampoos, conditioners, gels, pomades, cuticle coats, serum, sprays, colouring products and mascaras. The benefits of using the silicone aqueous dispersion in hair care compositions may include improved styling, fixative, conditioning, color retention and/or anti-frizz, and the benefits of using the silicone aqueous dispersion on eyelashes may include thickening, water resistance, and/or eyelash lengthening (extension). Nail care compositions include color coats, base coats, nail hardeners. The benefits of using the silicone aqueous dispersion in nail care compositions may include improved protection, long lasting effect, scratch resistance and/or adhesion. The aqueous silicone dispersion of the invention can be formulated into an oil in water cosmetic formulation or into a water in oil cosmetic formulation.

Health care compositions in which the dispersion of the invention can be used to deliver a pharmaceutically active ingredient include patches, creams, unguents, sticks, sprays and medicated nail varnish.

The aqueous silicone dispersion of the invention can also be used in cosmetic treatment without requiring an added cosmetically active ingredient. For example the aqueous silicone dispersion can be used to mask skin wrinkles. A method according to the invention of masking skin wrinkles in a mammal in need of such treatment comprises topically applying an effective amount of the aqueous silicone dispersion of the invention to the skin of the mammal.

There is a need for skin care products that combine beneficial properties such as concealing skin blemishes or wrinkles while leaving a natural look, providing good aesthetics together with a desirable sensory feel. Some known products have the power to mask imperfections, some give a natural look, and others offer a desirable sensory feel, but it has been difficult to achieve all of these attributes in a single product. A variety of cosmetic formulations on the market mask skin imperfections by concealing the natural skin. Such products are generally opaque or semi-opaque and deposit a non-transparent layer onto the skin, thereby concealing the natural skin tone and/or color. Although there are products available that are designed to precisely match a variety of different skin tones, it is nearly impossible to precisely match each individual skin tone. As a result, under certain lighting conditions, especially under natural daylight, the presence of the cosmetic formulation may be noticeable, which is undesirable. Moreover, each individual's skin has pores and other uneven areas. Formulations that are designed to conceal the natural appearance of the skin by depositing an opaque or semi-opaque layer onto the skin tend to fill those pores and uneven areas of the skin, which is also generally visible, especially under natural light or after several hours of wear. Further, especially in warmer and more humid climates, formulations that conceal the appearance of natural skin tend to either melt and deposit themselves into the skin's uneven areas (i.e., pores, wrinkles) or to have a caked appearance, both of which are undesirable. There is a need for products that maintain the natural look of the skin under a variety of lighting conditions.

We have found that the aqueous silicone dispersion of the invention comprising PVA can give a unique soft focus effect. Light reflected back from the skin is scattered. This hides skin imperfections such as wrinkles, crow's feet, signs of intrinsic and extrinsic skin aging and benign pigment disorders, tired and/or flabby skin, age spots, fatty and/or impure skin, and UV-damaged or irritated skin, while allowing the natural skin tones to be seen. The use of the silicone dispersion comprising PVA provides aesthetic benefits and comfort to the skin.

The term "soft focus" is used here to describe an optical effect that results in the object being seen by the observing eyes without sharp resolution. Thus, a cosmetic or skin care formulation which provides soft focus effect will, upon application to the skin, result in the natural skin being seen with the undesirable features such as wrinkles, pores, pigment spots, etc. being de-focused and not seen with sharp resolution. Therefore, the skin appears, to the naked eye, smoother, younger, and more even, while still looking natural. The soft focus effect differs from an opaque effect or appearance in that with soft focus, the light still reaches the skin and radiates back to the observing eye, although the light is diffused or scattered.

One of the ways to quantitatively assess such a soft focus effect is through measurement of light that is transmitted or reflected at a single specific angle versus scattered in different directions. A film of a sample material under consideration is first coated onto a glass slide by means of a bar applicator with a fixed gap. The gap distance determines the film thickness. This amount is representative of the usual amount of skin care formulation that one would apply to the skin. The film is then dried under ambient conditions to allow any volatile content to evaporate.

The glass slide with the coating is then placed in a spectrophotometer where an incident light is shone through the sample. The spectrophotometer is capable of measurement within a range of wavelengths (800-200 nm) corresponding to the visible and UV region. The incident light is partially transmitted, partially reflected and partially absorbed. The total transmittance, diffuse transmittance, total reflectance, and diffuse reflectance are measured. The total transmittance (TT) is the transmitted light collected within the half of the sphere forward of the sample. Diffuse transmittance (DT) is total transmittance minus the light collected within a forward solid angle of 8º around the incident light direction. The total reflectance (TR) is the reflected light collected within the half of the sphere backward of the sample. Light reflected at an angle equal to the incident angle is called specular reflection (SR). Diffuse reflectance (DR) is total reflectance minus specular reflection (DR = TR - SR). These quantities are typically expressed as a percentage of the incident light beam intensity. The amount of light absorbed (AB) is measured by subtracting the combined total transmittance (TT) and total reflectance (TR) from 100%. Thus, AB = 100 % - (TT+TR).

For a sample material to exhibit the property of soft focus, the amount of incident light that is absorbed by the sample material is below 25%. Most preferably the amount of incident light that is absorbed by the sample material is below 10%. A particularly desired soft focus effect of the compositions according to the present disclosure is one where DT is as high as possible with most of the light being transmitted instead of reflected or absorbed. When a large amount of light is absorbed by the composition, the skin will appear dim or dark, instead of radiant, which is undesirable. When most of the incident light is reflected off of the composition, the skin may appear bright but the reflection is from the composition rather than from the skin, so the natural skin is not being seen. A desirable effect is one that has a minimal amount of absorption by the composition and a relatively low amount of total reflection. A higher fraction of the diffused light from the light that is transmitted through or reflected from the composition leads to a blurrier effect; i.e., the skin appears more even. In other words, higher DT/TT and DR/TR ratios are more desirable. All the preceding measurements are based upon measurement on a film of specified thickness. A different film thickness may yield different measurements for the above parameters; for instance, the thicker the film, the lower the TT and the higher the AB.

When the aqueous silicone dispersion of the invention is thus used to mask skin imperfections, it advantageously contains solid particles, for example particles of silica, alumina, mica, clay, titanium dioxide, iron oxide, zinc oxide, boron nitride, zeolite, laponite, talc, silicone resins, silicone elastomer powders and their suspension , acrylic and acrylate homo- and co-polymers, nylon, polyethylene, colloidal metals, natural powders such as starch, or any combination thereof. The particulate solid may be untreated or can be any treated form of the above material; for example the particles may be surface treated for hydrophobization, hydrophilisation, and/or compatibilization. The particulate solid may have an average particle size of from 100 nanometers to 100 microns, alternatively of from 1 to 50 microns, alternatively of from 2 to 20 microns. The solid particles may have any shape, such as for example spherical, substantially spherical, hemispherical or irregular. The particles can be solid and impervious or may be porous or hollow particles. The aqueous silicone dispersion may also contain one or more waxes and/or fluids such as a silicone fluid, a hydrocarbon fluid or a blend of a silicone fluid or hydrocarbon fluid with a silicone gum.

The aqueous silicone dispersion of the invention can also be used in industrial coating applications. For example it can be coated on paper to give release properties.

The following comparative examples may help illustrate some aspects and advantages of this invention. They should not be interpreted as being prior art.

### Comparative Example C1

This Example is based on Example 1 of US-B-6306411. A solution of 105.11 g of deionized water and 3.97g of hydroxyethyl cellulose, supplied by Ashland, was prepared first. 64g of DOW CORNING® 9509 silicone elastomer suspension was weighed in a dental cup followed by the hydroxyethyl cellulose solution and placed into a Speedmixer® DAC 450 mixer and the cup was spun for 1 min at maximum speed. 12.93g of Avalure ® UR450 (what is this?) and 2g of glycerol were then added successively into the cup and spun for 1 min at maximum speed.

A film of 1mm is prepared out of the mixture prepared above with an Elcometer 3580 Casting Knife Film Applicator. The film was too weak and too brittle to perform mechanical resistance testing.

### Comparative Example C2

9.09g of Dow Corning® 9701 Cosmetic Powder (comprising Dimethicone / Vinyl Dimethicone Crosspolymer and Silica, and produced by the process described in patent JP-A-10/175816), was weighted in a dental cup followed by 6.03g of Mowiol 18-88 in solution at 18wt% and 11.25g of water. The cup was closed and placed into a Speedmixer® DAC 450 mixer and the cup was spun for 1 min at maximum speed.

A film of 1mm was prepared out of the mixture prepared above with an Elcometer 3580 Casting Knife Film Applicator. A film was initially formed and then became brittle and broke into hard pieces of flakes.

### Comparative Example C3

29.89g of a Vinyl-terminated functional linear siloxane having a viscosity of approximately 50,000 mPa.s was weighed into a dental cup followed by 0.167g of a hydride functional siloxane oligomer of 7 mPa.s containing 0.36wt% of hydride functions. The cup was closed and placed into a Speedmixer® DAC 450 mixer and the cup was spun for 1 min at maximum speed. 0.56g of polyoxyethylene (6) tridecyl ether, as nonionic surfactant, and 0.09g of Syloff 4000 catalyst (providing 15ppm of platinum) were added into the cup. The cup was closed and placed into a Speedmixer® DAC 450 mixer and the cup was spun for 1 min at maximum speed. 1.25g of deionized water was added into the cup and spun for 1 min at maximum speed. The concentrated emulsion thus formed was diluted incrementally with the amount of deionized water required to reach a total of 41.23g. The cup was stirred at maximum speed after each water addition for 1min.

Hydrosilylation took place in the emulsion and a film of 1mm was prepared from the resulting dispersion with an Elcometer 3580 Casting Knife Film Applicator the day after preparation. Table 1 lists the properties of the film. The film does not have mechanical resistance.

The invention is illustrated by the following Examples, in which parts and percentages are by weight unless otherwise stated. In the Examples, the quoted viscosity of the alkenyl-containing organopolysiloxanes is rotational viscosity measured at 23ºC following ASTM D 1084 with a Shibaura System KK, V type, No.4 at 6 rpm. The quoted viscosity of the SiH containing siloxanes is Glass Capillary Viscosity measured at 23ºC following ASTM D-445. The viscosity of the polyvinyl alcohol is measured as the viscosity of a 4 % aqueous solution at 20 °C determined by Hoppler viscometer (DIN 53015).

The mechanical resistance of the film produced according to the invention is evaluated by a texturometer. A 30cm diameter disc of film 1 mm thick previously deposited and stripped from its substrate is clamped between supports so that a portion of film of diameter 1cm is unsupported. A stainless steel spherical probe of diameter 5mm is contacted with the film. The probe moves downwards at a speed of 1.10 mm per second and when in contact with the film it goes down between 1.5 and 2 cm. The force and the distance required to break the film are recorded, and from these the breaking strength is calculated. The results are shown in Table 1. A breaking force of at least 200g and a breaking strength of at least 150 g.cm is considered to show an adequately strong film.

In many Examples, the aqueous silicone dispersion was applied to a glass microscope slide to form a film. The film was then left to dry for two hours at room temperature. A droplet of water was deposited onto the dried film and sixty seconds after the deposition, the contact angle (CA) of the water droplet with the film was measured.

### Example 1

64.96% of a Vinyl-terminated functional linear siloxane having a viscosity of approximately 50,000 mPa.s. (cPs) was weighed into a dental cup followed by 0.47% of a hydride functional siloxane oligomers of 7 mPa sec containing 0.36wt% of hydride functions. The cup was closed and placed into a Speedmixer® DAC 450 mixer and the cup was spun for 1 min at maximum speed. 6.7% of a 18% active solution of Mowiol 18-88 polyvinyl alcohol (88% hydrolysed, viscosity 18 mPa.s) and 0.2% of Syloff 4000 catalyst (providing 15ppm of platinum) were added into the cup. The cup was closed and placed into a Speedmixer® DAC 450 mixer and the cup was spun for 1 min at maximum speed. 1.6% of deionized water is added in the cup and spun for 1 min at maximum speed. The thick phase composition was diluted incrementally with the amount of deionized water required to reach a total of 100%. The cup was stirred at maximum speed after each water addition for 1min.

Hydrosilylation proceeded in the emulsion. A film 1 mm thick was prepared from the resulting dispersion with an Elcometer 3580 Casting Knife Film Applicator the day after preparation. Table 1 lists the properties of the film.

### Example 2

59.58% of a Vinyl-terminated functional linear siloxane having a viscosity of approximately 50,000 mPa.s was weighed into a dental cup followed by 0.40% of a hydride functional siloxane oligomer of 7 mPa.s containing 0.36wt% of hydride functions. The cup was closed and placed into a Speedmixer® DAC 450 mixer and the cup was spun for 1 min at maximum speed. 10.00% of a 18% active solution of Mowiol 18-88 and 0.13% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min.

### Examples 3 and 4

Example 2 was repeated using increasing amounts of the 18% active solution of Mowiol 18-88. The amount of this solution is respectively 15% and 30% in Examples 3 and 4.

In each of Examples 2 to 4 hydrosilylation took place in the emulsion. A film of 1mm was prepared from each resulting dispersion with an Elcometer 3580 Casting Knife Film Applicator the day after preparation. Table 1 lists the properties of the films. Table 1 shows that the dispersions of Examples 1 to 4 stabilized with polyvinyl alcohol have adequate mechanical resistance. Contrary to the comparative example C4, the film has a semi-hydrophilic behaviour, as shown by the contact angle measured.

### Examples 5 to 8

Example 2 was repeated using varying amounts of different grades of polyvinyl alcohol, as follows:
Example 5 - 20.0% of a 10% solution of Mowiol 20-98 (98% hydrolysed, viscosity 20 mPa.s)
Example 6 - 20.2% of a 10% solution of Mowiol 8-88 (88% hydrolysed, viscosity 8 mPa.s)
Example 7 - 19.7% of a 10% solution of Mowiol 30-88 (88% hydrolysed, viscosity 30 mPa.s)
Example 8 - 20.2% of a 10% solution of Mowiol 30-92 (92% hydrolysed, viscosity 30 mPa.s)

A film of 1mm was prepared from each resulting dispersion with an Elcometer 3580 Casting Knife Film Applicator the day after preparation. For each film, the contact angle was measured. As seen in Table 1, the nature of the polyvinyl alcohol can slightly impact the contact angle. However, the value indicates that the surface of the films stays hydrophilic to semi-hydrophilic.

### Examples 9 to 13

Example 2 was repeated using varying amounts of the hydride functional siloxane oligomer of 7 mPa.s containing 0.36wt% of hydride functions. For Examples 9, 10, 11, 12 and 13 the amount of hydride functional siloxane was respectively 0.47%, 0.51%, 0.57%, 0.30% and 0.81%. The molar ratio between SiH groups and vinyl groups was calculated for each Example and is shown in Table 1.

A film 1mm thick was prepared from each dispersion with an Elcometer 3580 Casting Knife Film Applicator the day after preparation. Table 1 lists the properties of each film. By varying the molar ratio of the alkenyl-containing organopolysiloxane (a) and the SiH containing siloxane (b) between 0.5 and 1.5, the overall mechanical properties vary slightly but all of Examples 9 to 13 produce adequately strong films.

### Example 14

59.58% of a Vinyl-terminated functional linear siloxane having a viscosity of approximately 50,000 mPa.s was weighed into a dental cup followed by 0.07% of a hydride functional siloxane oligomer of viscosity 30 mPa.s containing 1.66wt% of hydride functions. The cup was closed and placed into a Speedmixer® DAC 450 mixer and the cup was spun for 1 min at maximum speed. 10.00% of a 18% active solution of Mowiol 18-88 and 0.06% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min.

A film 1mm thick was prepared from each dispersion with an Elcometer 3580 Casting Knife Film Applicator the day after preparation. Table 1 lists the properties of the film. By changing the SiH containing siloxane (b) but maintaining the molar ratio between the Vinyl and SiH groups to 0.7, the mechanical resistance of the film is maintained.

**Table 1**

| Example Nº | Molar Ratio SiH/vinyl | Polyvinyl alcohol | | Force (g) | Distance (cm) | Breaking Strength (g.cm) | CA on glass (º) |
|---|---|---|---|---|---|---|---|
| | | Grade | Solution (wt%) | | | | |
| Comparative example C1 | | | | no film | | | |
| Comparative example C2 | | Mowiol 18-88 | 10.6 | no film - brittle powder | | | |
| Comparative example C3 | 0.6 | none | | 21.175 | 1.893 | 40.08 | |
| example 1 | 0.79 | Mowiol 18-88 | 6.7 | 239.277 | 1.199 | 288.07 | |
| example 2 | 0.74 | Mowiol 18-88 | 10.0 | 616.20 | 0.840 | 517.61 | 31.7 |
| example 3 | 0.76 | Mowiol 18-88 | 15.0 | 886.30 | 0.750 | 664.73 | 35.9 |
| example 4 | 0.72 | Mowiol 18-88 | 30.0 | 1527.20 | 0.620 | 946.86 | 46.3 |
| example 5 | 0.742 | Mowiol 20-98 | 20.0 | 696.70 | 0.610 | 424.99 | 77.1 |
| example 6 | 0.778 | Mowiol 8-88 | 20.2 | | | | 33.6 |
| example 7 | 0.779 | Mowiol 30-88 | 19.7 | | | | 39.1 |
| example 8 | 0.742 | Mowiol 30-92 | 20.2 | | | | 41.7 |
| example 9 | 0.86 | Mowiol 18-88 | 10.0 | 798.32 | 0.668 | 533.55 | |
| example 10 | 0.94 | Mowiol 18-88 | 10.0 | 849.13 | 0.639 | 542.31 | |
| example 11 | 1.04 | Mowiol 18-88 | 10.0 | 749.45 | 0.592 | 443.42 | |
| example 12 | 0.56 | Mowiol 18-88 | 10.0 | 519.00 | 0.860 | 446.34 | |
| Example 13 | 1.5 | Mowiol 18-88 | 10.0 | 628.00 | 0.957 | 601.00 | |
| Example 14 | 0.713 | Mowiol 18-88 | 10.0 | 442 | 0.871 | 385.4 | |

### Example 16

59.21% of a Vinyl-terminated functional linear siloxane having a viscosity of approximately 50,000 mPa.s was weighed into a dental cup followed by 0.41% of a hydride functional siloxane oligomers of 7 mPa.s containing 0.36wt% of hydride functions. The cup was closed and placed into a Speedmixer® DAC 450 mixer and the cup was spun for 1 min at maximum speed. 15% of a 18% active solution of Mowiol 18-88, 0.18% of polyoxyethylene (6) tridecyl ether and 0.2% of Syloff 4000 catalyst were also added into the cup. The weight ratio of PVA to nonionic surfactant was 15:1. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min.

### Example 17

59.15% of a Vinyl-terminated functional linear siloxane having a viscosity of approximately 50,000 mPa.s was weighed into a dental cup followed by 0.41% of a hydride functional siloxane oligomers of 7 mPa.s containing 0.36wt% of hydride functions. The cup was closed and placed into a Speedmixer® DAC 450 mixer and the cup was spun for 1 min at maximum speed. 14.7% of a 18% active solution of Mowiol 18-88 and 3.4% of a 30% active solution of cetyl ammonium chloride, as cationic surfactant and 0.2% of Syloff 4000 catalyst were also added into the cup. The weight ratio of PVA to nonionic surfactant was 2.7:1. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min.

Hydrosilylation took place in the emulsion and a film 1 mm thick was prepared from the resulting dispersion with an Elcometer 3580 Casting Knife Film Applicator the day after preparation. The mechanical resistance of the film prepared is listed in Table 1 and can be seen to be adequate. 1 wt% of cationic surfactant, in a weight ratio of 1:2.7 to the polyvinyl alcohol, can be added without impairing the resistance of the film.

Example 18: 59.60% of a Vinyl-terminated functional linear siloxane having a viscosity of approximately 50,000 mPa.s was weighed into a dental cup followed by 0.41% of a hydride functional siloxane oligomers of 7 mPa.s containing 0.36wt% of hydride functions. The cup was closed and placed into a Speedmixer® DAC 450 mixer and the cup was spun for 1 min at maximum speed. 37% of a 18% active solution of Mowiol 18-88 and 1% of Brij® 78, Polyoxyethylene (20) Stearyl Ether, as nonionic surfactant and 0.2% of Syloff 4000 catalyst were also added into the cup. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min.

Hydrosilylation took place in the emulsion and a film 1mm thick was prepared from the resulting dispersion with an Elcometer 3580 Casting Knife Film Applicator the day after preparation. The mechanical resistance of the film prepared is listed in Table 2 and can be seen to be adequate. The ratio of active between PVA and non-polymeric surfactant is at 6.7 which is high enough to get the appropriate resistance of the film.

Example 19: Example 18 was repeated replacing the 1% of Brij® 78 nonionic surfactant by 1% of Nacconol® 90G, Linear Alkylbenzene Sulfonate anionic surfactant. The mechanical resistance of the film prepared is listed in Table 2 and can be seen to be adequate. The ratio of active between PVA and non-polymeric surfactant is at 6.7 which is high enough to get the appropriate resistance of the film.

**Table 2**

| Example Nº | non-polymeric surfactant | PVA solution Concentrati on (wt%) | Amount of non-polymeric surfactant (wt%) | Active PVA/ Active su rfactant | Force (g) | Distance (cm) | breaking strength (g.cm) |
|---|---|---|---|---|---|---|---|
| Example 16 | Synperoni c 13/6 | 15.0 | 0.18 | 15 | 222.88 | 0.819 | 182.54 |
| Example 17 | Arquad 16-29 | 14.7 | 3.4 (1% active) | 2.7 | 217.79 4 | 0.984 | 214.285 |
| Example 18 | Brij 78 | 37 | 1 | 6.7 | 492.2 | 0.356 | 175.223 |
| Example 19 | Nacconol 90G | 37 | 1 | 6.7 | 739.4 | 0.3468 | 256.424 |

The influence of non-surfactant additives is shown in Examples 20 to 26 and the results are gathered in Table 3.

### Example 20

59.58% of a Vinyl-terminated functional linear siloxane having a viscosity of approximately 50,000 mPa.s was weighed into a dental cup followed by 0.40% of a hydride functional siloxane oligomer of 7 mPa.s containing 0.36wt% of hydride functions. The cup was closed and placed into a Speedmixer® DAC 450 mixer and the cup was spun for 1 min at maximum speed. 10.00% of a 18% active solution of Mowiol 18-88 and 0.13% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 90%. The cup was stirred at maximum speed after each water addition for 1 min. 10% glycerol was then added incrementally with the cup being stirred at maximum speed after each addition for 1min.

Hydrosilylation took place in the emulsion and a film 1 mm thick was prepared from the resulting dispersion with an Elcometer 3580 Casting Knife Film Applicator the day after preparation. The mechanical resistance of the film prepared is listed in Table 3. The glycerol is seen to have softened the film; the breaking force is lower than for Example 2, but the distance of deformation before breaking is higher.

### Examples 21 and 22

Example 20 was repeated replacing the glycerol by a mixture of capric and caprylic tryglicerides (Example 21) or petrolatum (Example 22). The mechanical resistance of the films prepared is listed in Table 3 and is seen to be adequate.

### Example 23

59.58% of a Vinyl-terminated functional linear siloxane having a viscosity of approximately 50,000 mPa.s was weighed into a dental cup followed by 0.40% of a hydride functional siloxane oligomer of 7 mPa.s containing 0.36wt% of hydride functions. The cup was closed and placed into a Speedmixer® DAC 450 mixer and the cup was spun for 1 min at maximum speed. 10.00% of a 18% active solution of Mowiol 18-88 and 0.13% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 94%. The cup was stirred at maximum speed after each water addition for 1 min. 6% polyethylene glycol PEG400 (molecular weight 400) was then added incrementally with the cup being stirred at maximum speed after each addition for 1min.

Hydrosilylation took place in the emulsion and a film 1 mm thick was prepared from the resulting dispersion with an Elcometer 3580 Casting Knife Film Applicator the day after preparation. The mechanical resistance of the film prepared is listed in Table 3 and is seen to be adequate.

Glycerol, capric/caprylic tryglicerides, petrolatum and PEG400 were chosen as examples of materials which may be used as excipients for pharmaceutically or cosmetically active ingredients. Such excipients can be included in the dispersions and multilayer films of the invention without substantially affecting the physical properties of the films.

### Example 24

59.58% of a Vinyl-terminated functional linear siloxane having a viscosity of approximately 50,000 mPa.s was weighed into a dental cup followed by 0.40% of a hydride functional siloxane oligomer of 7 mPa.s containing 0.36wt% of hydride functions and 10% of Dow Corning ® 200 fluid 350, a trimethylsilyl-endblocked polydimethylsiloxane of kinetic viscosity 350 cSt. The cup was closed and placed into a Speedmixer® DAC 450 mixer and the cup was spun for 1 min at maximum speed. 15.00% of a 18% active solution of Mowiol 18-88 and 0.13% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min.

### Example 25

59.58% of a Vinyl-terminated functional linear siloxane having a viscosity of approximately 50,000 mPa.s was weighed into a dental cup followed by 0.40% of a hydride functional siloxane oligomer of 7 mPa.s containing 0.36wt% of hydride functions and 5% of Dow Corning ® 200 fluid 10000, a trimethylsilyl-endblocked polydimethylsiloxane of kinetic viscosity 10000 cSt. The cup was closed and placed into a Speedmixer® DAC 450 mixer and the cup was spun for 1 min at maximum speed. 10.00% of a 18% active solution of Mowiol 18-88 and 0.13% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min.

In each of Examples 24 and 25 hydrosilylation took place in the emulsion and a film 1mm thick was prepared from the resulting dispersion with an Elcometer 3580 Casting Knife Film Applicator the day after preparation. The mechanical resistance of the films prepared is listed in Table 3 and is seen to be adequate. The polydimethylsiloxanes, which remain in the silicone phase but do not react, are seen to have modified the film; the breaking force is lower than for Example 3, but the distance of deformation before breaking is higher.

### Example 26

59.58% of a Vinyl-terminated functional linear siloxane having a viscosity of approximately 50,000 mPa.s was weighed into a dental cup followed by 0.40% of a hydride functional siloxane oligomer of 7 mPa.s containing 0.36wt% of hydride functions. The cup was closed and placed into a Speedmixer® DAC 450 mixer and the cup was spun for 1 min at maximum speed. 10.00% of a 18% active solution of Mowiol 18-88 and 0.13% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 95.98%. The cup was stirred at maximum speed after each water addition for 1min. 4.02% Dow Corning® 9701 Cosmetic Powder (comprising Dimethicone / Vinyl Dimethicone crosspolymer and silica) was then added with the cup being stirred at maximum speed for 1 min. after addition of the powder.

Hydrosilylation took place in the emulsion and a film 1 mm thick was prepared from the resulting dispersion with an Elcometer 3580 Casting Knife Film Applicator the day after preparation. The mechanical resistance of the film prepared is listed in Table 3 and is seen to be adequate. The film was soft and pleasant to the touch. The process of the present invention is thus an effective method of applying the cosmetic powder of Comparative Example 2 in the form of a soft film.

**Table 3**

| Example Nº | Additive | Molar Ratio SiH/Vinyl | PVA solution Concentr ation (wt%) | Amount of additive (wt%) | Force (g) | Distance (cm) | Breaking strength (g.cm) |
|---|---|---|---|---|---|---|---|
| example 20 | Glycerin | 0.76 | 10.00 | 10.00 | 185.58 | 1.35 | 249.73 |
| example 21 | Capric/C aprylic Triglyceri de | 0.79 | 10.00 | 10.00 | 571.13 | 0.67 | 380.75 |
| example 22 | Petrolatu m | 0.78 | 10.00 | 10.00 | 446.94 | 0.58 | 258.33 |
| example 23 | PEG400 | 0.79 | 10.00 | 6.00 | 357.95 | 0.84 | 301.03 |
| example 24 | 200 fluid 350 Cst | 0.740 | 15.00 | 10.00 | 571.11 | 0.81 | 464.88 |
| example 25 | 200 fluid 10000 Cst | 0.760 | 15.00 | 5.00 | 681.15 | 0.89 | 608.95 |
| example 26 | 4% 9701 cosmetic powder | 0.76 | 10.00 | | 539.27 | 0.58 | 314.40 |

### Example 27

85.04% of a Vinyl-terminated functional linear siloxane having a viscosity of approximately 50,000 mPa.s was weighed into a dental cup followed by 0.62% of a hydride functional siloxane oligomers of 7 mPa.s containing 0.36wt% of hydride functions. The cup was closed and placed into a Speedmixer® DAC 450 mixer and the cup was spun for 1 min at maximum speed. 14.27% of a 18% active solution of Mowiol 18-88 and 0.07% of Syloff 4000 catalyst were added into the cup. The cup was closed and placed into a Speedmixer® DAC 450 mixer and the cup was spun for 1 min at maximum speed.

The above composition was diluted incrementally with a solution of deionized water, caffeine and pentylene glycol. The final composition contains 70% of the above emulsion, 26.52% of deionized water, 2.28% of pentylene glycol and 1.20% of caffeine. The cup was stirred at maximum speed after each addition for 1min. Hydrosilylation took place in the silicone droplets of the emulsion.

### Example 28

79.36% of a Vinyl-terminated functional linear siloxane having a viscosity of approximately 50,000 mPa.s was weighed into a dental cup followed by 0.56% of a hydride functional siloxane oligomers of 7 mPa.s containing 0.36wt% of hydride functions. The cup was closed and placed into a Speedmixer® DAC 450 mixer and the cup was spun for 1 min at maximum speed. 20.02% of a 18% active solution of Mowiol 18-88 and 0.07% of Syloff 4000 catalyst were added into the cup. The cup was closed and placed into a Speedmixer® DAC 450 mixer and the cup was spun for 1 min at maximum speed.

The above composition was diluted incrementally with a solution of deionized water, caffeine and pentylene glycol. The final composition contains 75% of the above emulsion, 21.52% of deionized water, 2.28% of pentylene glycol and 1.20% of caffeine. The cup was stirred at maximum speed after each addition for 1min. Hydrosilylation took place in the silicone droplets of the emulsion.

The dispersions of Examples 27 and 28 were tested using the following protocol: Skin preparation

Porcine ears were obtained from a local slaughterhouse and cleaned under cold running water. Skin was excised with a scalpel and cut to a thickness of 750 µm with an electric dermatome (Zimmer™ Electric Dermatome, Dover, Ohio). The skin was cut into ∼9 cm² pieces, which were individually wrapped in Parafilm™ and kept at -20 ºC for a maximum of 3 months before use.

To check the quality of the porcine skin samples, a skin integrity test was performed prior to the penetration experiment. The skin integrity is tested by measuring TEWL (TransEpidermal Water Loss) *in vitro* (with the Aquaflux equipment) after having mounted the dermatomed pig skin on the cell and after having waited for 20 min of equilibration. A maximum threshold value of 12.0 g.m⁻².h⁻¹ is the pass-fail criteria for intact skin. If the TEWL *in vitro* of the dermatomed pig skin sample is above this threshold value, it is then removed and replaced by a new one.

*In vitro* skin penetration were carried out on the Logan automatic Franz cell equipment under the following conditions:
- Cell size: internal diameter = 15 mm, penetration surface = 1.77 cm², receptor volume ∼12 ml, individual calibration as per.
- Receptor solution and type: Phosphate Buffered Saline, pH 7.4
- Sampling parameters:
   ∘ Flush 5.0 mL
   ∘ Waste 1.0 mL
   ∘ Sample 1.0 mL
   ∘ Return to waste: yes
   ∘ Replacement volume: 10.2 mL
- Amount of formulation deposited on the membrane: determined gravimetrically by differential weight, ranging from ∼50 to ∼70 mg per cell
- Type of membrane: dermatomed pig skin (750 µm)
- Test temperature: 32 ºC
- Sampling schedule: 1, 2, 3, 4, 5, 6 hours

For each formulation, the testing was carried out in 4 replicates. For each formulation, each replicate represented skin penetration from a different donor (pig skin). After application of the formulation, the cell tops of the compartment were covered with Parafilm to avoid evaporation and ensure consistent test conditions across all formulations.

Around 0.1 g of sample is weighted accurately and dissolved in 5 mL of toluene in a 50 mL polyethylene centrifuge tube. The tube is vortexed and mechanically agitated for 1 h before extraction of the caffeine with 40 mL of ultra pure water (accurately weighted). The centrifuge tube is shacked during 15 min before centrifugation (10 min) at 4,000 RPM (Revolution Per Minute) or 2,000 RCF (Relative Centrifuge Force). The silicone contained in the formulation is precipitated in the toluene phase and the caffeine is extracted in the aqueous phase. Finally, the aqueous phase is carefully filtered using 0.2 µL regenerated cellulose filter in 2 mL HPLC (high performance liquid chromatography) glass autosampler vial to provide a sample for UPLC (ultra performance liquid chromatography) analysis for caffeine.

The UPLC method uses a Waters BEH C18 reverse phase column packed with trifunctional C18 carbon chain bonded to Ethylene Bridged Hybrid (BEH) substrates of 1.7 µm (spherical particle size) conferring very good stability even in a extreme pH range (from 1 to 12). This packing material (18% carbon load) allows the separation of a broad range of compound classes.

UPLC instrumental conditions were:

| | |
|---|---|
| LC system: | Waters ACQUITY UPLC® system |
| Detection: | Photodiode Array Detector 271 nm (resolution 4.8 nm) |
| Column: | ACQUITY UPLC BEH C18, 1.7 µm, 130 Å, 2.1 x 50 mm |
| Column temperature: | 40 °C (+/- 1°C) |
| Sample temperature: | 20 °C (+/- 1°C) |
| Flow rate: | 0.5 mL/min - Isocratic → 60% A - 40% B |
| Mobile phase A: | Water (Millipore DirectQ ultra pure water) - 18.2 MQ.cm |
| Mobile phase B: | Methanol - ULC/MS grade from Biosolve |
| Weak needle wash: | 60:40 (v/v) water/methanol (1000 µL) |
| Strong needle wash: | 1:1 (v/v) water/acetonitrile (500 µL) |
| Isocratic run time: | 1 min |
| Injection volume: | 3 µL, partial loop with needle overfill |

This method has been validated in terms of linearity with regression coefficient above 0.9999, precision with a relative standard deviation (RSD) below 1%. The limit of detection and quantification of caffeine in aqueous solution have been determined and are respectively 19 and 64 ppb (weight by weight). Results for the skin penetration samples of Examples 27 and 28 are shown in Table 4.

**Table 4**

| Sample | Cumulative drug passed (µg/cm²) | | Drug passed (% from initial dose) | |
|---|---|---|---|---|
| | Average | STDEV | Average | STDEV |
| Example 27 | 13.2 | 11.5 | 3.0 | 2.8 |
| Example 28 | 7.1 | 2.7 | 1.8 | 0.9 |

As seen in Table 4, the dispersions of Examples 27 and 28 were able to deliver noticeable amounts of caffeine through the skin - respectively 13.2 and 7.1 µg/cm² - and hence can serve as a vehicle for active pharmaceutical ingredients as well as cosmetic actives.

### Example 29

59.59% of a Vinyl-terminated functional linear siloxane having a viscosity of approximately 50,000 mPa.s was weighed into a dental cup followed by 1.17% of a linear hydride functional siloxane oligomer of 10 mPa.s containing 0.18wt% of hydride functions. The cup was closed and placed into a Speedmixer® DAC 450 mixer and the cup was spun for 1 min at maximum speed. 10.00% of a 18% active solution of Mowiol 18-88 and 0.05% of Syloff 4000 catalyst were added into the cup. The cup was closed and placed into a Speedmixer® DAC 450 mixer and the cup was spun for 1 min at maximum speed. The thick phase composition was diluted incrementally with the amount of deionized water required to reach a total of 100%. The cup was stirred at maximum speed after each water addition for 1min. A dispersion of a hydrosilylation reaction product was formed.

The dispersions of Example 1 and of Example 29 were each formulated into a water-in oil formulation having the composition shown in Table 5

**Table 5**

| **Ingredients** | **%** | **%** |
|---|---|---|
| | | |
| Phase A= oil phase | | |
| PEG-10 Dimethicone copolymer emulsifier | 2.00 | 2.00 |
| Cyclopentasiloxane and Dimethiconol silicone gum blend | 3.50 | 3.50 |
| Pentaerythrityl Tetraisostearate | 4.10 | 4.10 |
| Dimethicone Fluid, 1.5 cst | 12.40 | 12.40 |

| Phase B | | |
|---|---|---|
| Example 1 dispersion | 3.00 | - |
| Example 29 dispersion | - | 3.20 |
| Water | 75.00 | 74.80 |

The phase A ingredients were mixed together until homogeneous. The phase B ingredients were mixed together until homogeneous and were slowly added to phase A while mixing at 500-1000 rpm until homogeneous. When all phase B was added, the water-in-oil formulation was mix for an additional 5 minutes under high shear at 2000 rpm.

The water-in-oil formulations were applied to the skin of test panellists, and were also coated onto a glass slide at a thickness of 37 microns and assessed for soft focus using a PerkinElmer Lambda 950 UV-Vis Spectrophotometer equipped with a 150 mm Integrating sphere, at a wavelength of 550 nm. The spectrophotometer measurements were taken 15 minutes after coating.

The water-in-oil formulations of the dispersions of Examples 1 and 29 provided some wrinkle masking /blur effect while applied on the skin of the hand, as judged by simple visual assessment.

For the formulation based on Example 1, the total transmission TT was 92% and the total reflection TR was 7.5%. The ratio of diffuse transmission DT/TT was 20% and the ratio of diffuse reflection DR/TR was 40%. For the formulation based on Example 29, the total transmission TT was 91% and the total reflection TR was 7.5%. The ratio of diffuse transmission DT/TT was 25% and the ratio of diffuse reflection DR/TR was 49%. The TT and TR percentages in both Examples indicate good visibility of the natural skin tone. The values of DT/TT and DR/TR were however lower than the values usually required to give a substantial wrinkle masking effect.

The water-in-oil formulations of the dispersions of Examples 1 and 29 were each applied to the skin of test panellists around the eye area where the deep and fine wrinkles are seen. A picture is taken with a 'Visia' (trade mark) image analysis system before applying the product on the panellist skin, then a picture is taken just after product application (reference time 0) and respectively after 15 minutes and 6 hours. For Example 1 the pictures show a decrease the number of wrinkles just after product application but the number of wrinkles increases after 15 minutes, and after 6 hours no wrinkle masking effect is demonstrated. For Example 29 the pictures show a decrease the number of wrinkles just after product application and a further decrease after 15 minutes. After 6 hours the number of wrinkles increases a little but is still lower than the number of wrinkles before product application.

### Examples 30 to 36

Following the procedure of Claim 1, silicone dispersions were prepared from the reagents shown in Table 6. The Si-Vinyl Polymer was a vinyl-terminated functional linear siloxane having a viscosity of approximately 50,000 mPa.s. The SI-H Polymer was a hydride functional siloxane oligomers of viscosity 7 mPa.s containing 0.36wt% of hydride functions (%). After preparation of the dispersions, solid silicone particles were dispersed therein. The solid silicone particles each comprised a dimethicone/vinyl dimethicone crosspolymer. In one type of particle this crosspolymer is blended with silica. In the other type of particle the crosspolymer is blended with C12-14 pareth-12, a polyethylene glycol ether of a mixture of synthetic secondary C12-14 fatty alcohols with an average of 12 moles of ethylene oxide. The median particle size of the dispersions of Examples 31 to 35 containing the solid silicone particles was measured and is shown in Table 6.

**Table 6**

| Example | 30 | 31 | 32 | 33 | 34 | 35 | 36 |
|---|---|---|---|---|---|---|---|
| Si-Vinyl polymer (%) | 60.71 | 60.75 | 60.71 | 59.55 | 59.5 | 59.63 | 59.43 |
| Si-H polymer (%) | 0.43 | 0.43 | 0.43 | 0.42 | 0.43 | 0.41 | 0.42 |
| Mowiol 20-98 (%) | 18.34 | 18.43 | 18.42 | 0 | 0 | 0 | 0 |
| Mowiol 8-88 (%) | 0 | 0 | 0 | 17.97 | 18 | 18.04 | 18.05 |
| Syloff 4000 catalyst (%) | 0.12 | 0.12 | 0.14 | 0.14 | 0.14 | 0.12 | 0.14 |
| Water (%) | 15.2 | 10.02 | 5.03 | 17.93 | 15.9 | 12.41 | 6.94 |
| Particles of Dimethicone/Vinyl Dimethicone Crosspolymer and Silica (%) | 0 | 0 | 0 | 3.98 | 6.03 | 9.39 | 0 |
| Particles of Dimethicone/Vinyl Crosspolymer (and) C12-14 Pareth-12 (%) | 5.2 | 10.26 | 15.28 | 0 | 0 | 0 | 15.02 |
| % powder in dry film | 4.74% | 9.05% | 13.07% | 5.76% | 8.52% | 12.76% | 19.24% |
| Dv 0.5 (um) | | 6.344 | 6.573 | 4.786 | 5.955 | 5.634 | |

The dispersions of Examples 30 to 34 were each coated onto a glass slide at a thickness of 37 microns and assessed for soft focus using a PerkinElmer Lambda 950 UV-Vis Spectrophotometer equipped with a 150 mm Integrating sphere, at a wavelength of 550 nm. The spectrophotometer measurements were taken 15 minutes after coating and again 3 hours after coating.

For each of the dispersions of Examples 30 to 34 the TT after 15 minutes and also after 3 hours was 91 to 92% and the total reflection TR was 8%. The TT and TR percentages in all these Examples indicate good visibility of the natural skin tone. The ratios of diffuse transmission DT/TT and of diffuse reflection DR/TR measured are shown in Table 7.

**Table 7**

| Example | 30 | 31 | 32 | 33 | 34 |
|---|---|---|---|---|---|
| DT/TT 15 m | 70% | 62% | 54% | 47% | 50% |
| DR/TR 15 m | 86% | 81% | 81% | 70% | 72% |
| DT/TT 3 h | 69% | 58% | 52% | 48% | 50% |
| DR/TR 3 h | 85% | 76% | 60% | 73% | 77% |

The values of DT/TT and DR/TR after 15 minutes and after 3 hours were values indicating a substantial wrinkle masking effect, with the values achieved in Example 30 being particularly good.

## Claims

1. A process for the preparation of an aqueous silicone dispersion, the process comprising
mixing in a first step (a) an alkenyl-containing organopolysiloxane having an average per molecule of at least 2 alkenyl groups and (b) an SiH containing siloxane having an average per molecule of at least 2 SiH moieties, and
then emulsifying the resulting mixture in an aqueous polyvinyl alcohol solution to form an aqueous silicone emulsion ,
wherein a hydrosilylation catalyst is added simultaneously with the addition of the aqueous polyvinyl alcohol ,
the alkenyl-containing organopolysiloxane (a) and the SiH containing siloxane (b) being reacted together in the aqueous silicone emulsion, and the alkenyl-containing organopolysiloxane (a) and the SiH containing siloxane (b) and their reaction product being stabilised in dispersion form by the polyvinyl alcohol dissolved in the aqueous phase,
wherein the hydrosilylation reaction takes place within the disperse phase to form a soft elastomer and
wherein the aqueous silicone dispersion is a 1-part silicone elastomer emulsion.

2. A process according to Claim 1 wherein the alkenyl-containing organopolysiloxane has a dynamic viscosity of from 100 to 100,000,000 milliPascal-seconds (mPa.s) when tested as described in ASTM D1084 at 25°C.

3. A process according to Claim 1 wherein the alkenyl-containing organopolysiloxane is a vinyl-endcapped polydimethylsiloxane of formula CH₂=CH-Si(CH₃)₂O-[Si(CH₃)₂O]ₙ-Si(CH₃)₂CH=CH₂, wherein n is an average number of from 100 to 10000.

4. A process according to any of Claims 1 to 3 wherein an excipient oil is mixed with the alkenyl-containing organopolysiloxane (a) and the SiH containing siloxane (b) before the mixture of alkenyl-containing organopolysiloxane (a) and SiH containing siloxane (b) is emulsified.

5. A process according to any of Claims 1 to 4 wherein the aqueous silicone dispersion is prepared in the absence of any non-polymeric surfactant.

6. A pharmaceutical or cosmetic composition comprising an admixture of a dispersion and a pharmaceutically or cosmetically active ingredient, respectively,
wherein the dispersion comprises a silicone composition dispersed in an aqueous phase, the silicone composition comprising a product of a reaction of (a) an alkenyl-containing organopolysiloxane having an average per molecule of at least 2 alkenyl groups and (b) an SiH containing siloxane having an average per molecule of at least 2 SiH moieties, the dispersion composition also comprising a hydrosilylation catalyst and polyvinyl alcohol, the alkenyl-containing organopolysiloxane (a) and the SiH containing siloxane (b) being partially reacted together, and the silicone composition being stabilised in dispersion form by the polyvinyl alcohol dissolved in the aqueous phase, wherein the dispersion lacks any non-polymeric surfactant.

7. The pharmaceutical or cosmetic composition of claim 6 wherein the aqueous polyvinyl alcohol solution contains a non-ionic surfactant at a weight ratio of polyvinyl alcohol to nonionic surfactant of at least 5:1, or contains a cationic or anionic surfactant at a weight ratio of polyvinyl alcohol to cationic or anionic surfactant of at least 2.5:1.

8. A pharmaceutically or cosmetically active multilayer film comprising a multilayer film comprising a silicone layer in operative contact with a poly(vinyl alcohol) layer, the silicone layer comprising an aggregation of the disperse phase of the dispersion and the poly(vinyl alcohol) layer being characterizable by a water contact angle (θ) of less than 80 degrees (°) when θ is measured according to ASTM D7334-08 30 seconds after a test water droplet was deposited thereon and further containing a pharmaceutically or cosmetically active ingredient, respectively.

9. The pharmaceutically or cosmetically active multilayer film of claim 8 wherein the said silicone layer is an elastomeric silicone material formed by reaction of the alkenyl-containing organopolysiloxane (a) and the SiH containing siloxane (b).

10. A method of masking skin wrinkles in a mammal in need of such treatment, the method comprising topically applying an effective amount of a dispersion to the skin of the mammal
wherein the dispersion comprises a silicone composition dispersed in an aqueous phase, the silicone composition comprising a product of a reaction of (a) an alkenyl-containing organopolysiloxane having an average per molecule of at least 2 alkenyl groups and (b) an SiH containing siloxane having an average per molecule of at least 2 SiH moieties, the dispersion composition also comprising a hydrosilylation catalyst and polyvinyl alcohol, the alkenyl-containing organopolysiloxane (a) and the SiH containing siloxane (b) being partially reacted together, and the silicone composition being stabilised in dispersion form by the polyvinyl alcohol dissolved in the aqueous phase, wherein the dispersion lacks any non-polymeric surfactant.

## Patentansprüche

1. Ein Verfahren für die Herstellung einer wässrigen Silicondispersion, wobei das Verfahren Folgendes beinhaltet:
in einem ersten Schritt, Mischen (a) eines alkenylhaltigen Organopolysiloxans mit durchschnittlich mindestens 2 Alkenylgruppen pro Molekül und (b) eines SiH-haltigen Siloxans mit durchschnittlich mindestens 2 SiH-Anteilen pro Molekül und
dann Emulgieren der resultierenden Mischung in einer wässrigen Polyvinylalkohollösung, um eine wässrige Siliconemulsion zu bilden,
wobei ein Hydrosilylierungskatalysator zeitgleich mit der Zugabe des wässrigen Polyvinylalkohols zugegeben wird,
das alkenylhaltige Organopolysiloxan (a) und das SiH-haltige Siloxan (b) in der wässrigen Siliconemulsion miteinander zur Reaktion gebracht werden und das alkenylhaltige Organopolysiloxan (a) und das SiH-haltige Siloxan (b) und ihr Reaktionsprodukt durch den in der wässrigen Phase gelösten Polyvinylalkohol in Dispersionsform stabilisiert werden,
wobei die Hydrosilylierungsreaktion innerhalb der dispersen Phase stattfindet, um ein weiches Elastomer zu bilden, und
wobei die wässrige Silicondispersion eine 1-Teil-Siliconelastomeremulsion ist.

2. Verfahren gemäß Anspruch 1, wobei das alkenylhaltige Organopolysiloxan bei 25 °C beim Testen, wie in ASTM D1084 beschrieben, eine dynamische Viskosität von 100 bis 100 000 000 Millipascalsekunden (mPa.s) aufweist.

3. Verfahren gemäß Anspruch 1, wobei das alkenylhaltige Organopolysiloxan ein vinylendverkapptes Polydimethylsiloxan der folgenden Formel ist: CH₂=CH-Si(CH₃)₂O-[Si(CH₃)₂O]ₙ-Si(CH₃)₂-CH=CH₂, wobei n eine Durchschnittszahl von 100 bis 10 000 ist.

4. Verfahren gemäß einem der Ansprüche 1 bis 3, wobei ein Hilfsstoff-Öl mit dem alkenylhaltigen Organopolysiloxan (a) und dem SiH-haltigen Siloxan (b) gemischt wird, bevor die Mischung aus alkenylhaltigem Organopolysiloxan (a) und SiH-haltigem Siloxan (b) emulgiert wird.

5. Verfahren gemäß einem der Ansprüche 1 bis 4, wobei die wässrige Silicondispersion in Abwesenheit jeglichen nicht polymeren Tensids hergestellt wird.

6. Eine pharmazeutische oder kosmetische Zusammensetzung, beinhaltend ein Gemisch aus einer Dispersion und einem pharmazeutisch bzw. kosmetisch aktiven Inhaltsstoff, wobei die Dispersion eine in einer wässrigen Phase dispergierte Siliconzusammensetzung beinhaltet, wobei die Siliconzusammensetzung ein Produkt einer Reaktion von (a) einem alkenylhaltigen Organopolysiloxan mit durchschnittlich mindestens 2 Alkenylgruppen pro Molekül und (b) einem SiH-haltigen Siloxan mit durchschnittlich mindestens 2 SiH-Anteilen pro Molekül beinhaltet, wobei die Dispersionszusammensetzung auch einen Hydrosilylierungskatalysator und Polyvinylalkohol beinhaltet, wobei das alkenylhaltige Organopolysiloxan (a) und das SiH-haltige Siloxan (b) teilweise miteinander zur Reaktion gebracht werden und die Siliconzusammensetzung durch den in der wässrigen Phase gelösten Polyvinylalkohol in Dispersionsform stabilisiert wird, wobei die Dispersion kein nicht polymeres Tensid aufweist.

7. Pharmazeutische oder kosmetische Zusammensetzung gemäß Anspruch 6, wobei die wässrige Polyvinylalkohollösung ein nichtionisches Tensid mit einem Gewichtsverhältnis von Polyvinylalkohol zu nichtionischem Tensid von mindestens 5 : 1 enthält oder ein kationisches oder anionisches Tensid mit einem Gewichtsverhältnis von Polyvinylalkohol zu kationischem oder anionischem Tensid von mindestens 2,5 : 1 enthält.

8. Ein pharmazeutisch oder kosmetisch aktiver Mehrschichtfilm, beinhaltend einen Mehrschichtfilm, beinhaltend eine Siliconschicht in funktionsfähigem Kontakt mit einer Poly(vinylalkohol)schicht, wobei die Siliconschicht eine Aggregation der dispersen Phase der Dispersion beinhaltet und die Poly(vinylalkohol)schicht durch einen Wasserkontaktwinkel (θ) von weniger als 80 Grad (°) charakterisierbar ist, wenn θ gemäß ASTM D7334-08 30 Sekunden nach Ablagerung eines Testwassertropfens darauf gemessen wurde, und ferner enthaltend einen pharmazeutisch bzw. kosmetisch aktiven Inhaltsstoff.

9. Pharmazeutisch oder kosmetisch aktiver Mehrschichtfilm gemäß Anspruch 8, wobei die Siliconschicht ein elastomeres Siliconmaterial ist, gebildet durch eine Reaktion des alkenylhaltigen Organopolysiloxans (a) und des SiH-haltigen Siloxans (b).

10. Ein Verfahren zum Kaschieren von Hautfalten bei einem Säugetier, das einer solchen Behandlung bedarf, wobei das Verfahren das topische Auftragen einer wirksamen Menge einer Dispersion auf die Haut des Säugetiers beinhaltet, wobei die Dispersion eine in einer wässrigen Phase dispergierte Siliconzusammensetzung beinhaltet, wobei die Siliconzusammensetzung ein Produkt einer Reaktion von (a) einem alkenylhaltigen Organopolysiloxan mit durchschnittlich mindestens 2 Alkenylgruppen pro Molekül und (b) einem SiH-haltigen Siloxan mit durchschnittlich mindestens 2 SiH-Anteilen pro Molekül beinhaltet, wobei die Dispersionszusammensetzung auch einen Hydrosilylierungskatalysator und Polyvinylalkohol beinhaltet, wobei das alkenylhaltige Organopolysiloxan (a) und das SiH-haltige Siloxan (b) teilweise miteinander zur Reaktion gebracht werden und die Siliconzusammensetzung durch den in der wässrigen Phase gelösten Polyvinylalkohol in Dispersionsform stabilisiert wird, wobei die Dispersion kein nicht polymeres Tensid aufweist.

## Revendications

1. Un procédé pour la préparation d'une dispersion aqueuse de silicone, le procédé comprenant
le mélange dans une première étape (a) d'un organopolysiloxane contenant un alcényle ayant une moyenne par molécule d'au moins 2 groupes alcényle et (b) d'un siloxane contenant de la SiH ayant une moyenne par molécule d'au moins 2 groupes fonctionnels SiH, et
puis la mise en émulsion du mélange qui en résulte dans une solution d'alcool polyvinylique aqueux pour former une émulsion aqueuse de silicone,
dans lequel un catalyseur d'hydrosilylation est ajouté simultanément avec l'ajout de l'alcool polyvinylique aqueux,
l'organopolysiloxane contenant de l'alcényle (a) et le siloxane contenant de la SiH (b) étant mis à réagir ensemble dans l'émulsion aqueuse de silicone, et l'organopolysiloxane contenant de l'alcényle (a) et le siloxane contenant de la SiH (b) et leur produit de réaction étant stabilisés sous forme de dispersion par l'alcool polyvinylique dissous dans la phase aqueuse,
dans lequel la réaction d'hydrosilylation a lieu au sein de la phase dispersée pour former un élastomère souple et
dans lequel la dispersion aqueuse de silicone est une émulsion élastomère à 1 partie de silicone.

2. Un procédé selon la revendication 1 dans lequel l'organopolysiloxane contenant de l'alcényle a une viscosité dynamique allant de 100 à 100 000 000 millipascals-secondes (mPa.s) quand il est analysé tel que décrit dans ASTM D1084 à 25 °C.

3. Un procédé selon la revendication 1 dans lequel l'organopolysiloxane contenant de l'alcényle est un polydiméthylsiloxane coiffé à l'extrémité par un vinyle, de formule CH₂=CH-Si(CH₃)₂O-[Si(CH₃)₂O]ₙ-Si(CH₃)₂-CH=CH₂, dans lequel n est un nombre moyen allant de 100 à 10 000.

4. Un procédé selon n'importe lesquelles des revendications 1 à 3 dans lequel une huile d'excipient est mélangée avec l'organopolysiloxane contenant de l'alcényle (a) et le siloxane contenant de la SiH (b) avant que le mélange d'organopolysiloxane contenant de l'alcényle (a) et du siloxane contenant de la SiH (b) soit émulsionné.

5. Un procédé selon n'importe lesquelles des revendications 1 à 4 dans lequel la dispersion aqueuse de silicone est préparée en l'absence de tout tensioactif non polymérique.

6. Une composition pharmaceutique ou cosmétique comprenant un mélange additionnel d'une dispersion et d'un ingrédient pharmaceutiquement ou cosmétiquement actif, respectivement,
dans lequel la dispersion comprend une composition de silicone dispersée dans une phase aqueuse, la composition de silicone comprenant un produit d'une réaction (a) d'un organopolysiloxane contenant de l'alcényle ayant une moyenne par molécule d'au moins 2 groupes alcényle et (b) d'un siloxane contenant de la SiH ayant une moyenne par molécule d'au moins 2 groupes fonctionnels SiH, la composition de dispersion comprenant aussi un catalyseur d'hydrosilylation et de l'alcool polyvinylique, l'organopolysiloxane contenant de l'alcényle (a) et le siloxane contenant de la SiH (b) étant partiellement mis à réagir ensemble, et la composition de silicone étant stabilisée sous forme de dispersion par l'alcool polyvinylique dissous dans la phase aqueuse, dans lequel la dispersion est exempte de tout tensioactif non polymérique.

7. La composition pharmaceutique ou cosmétique de la revendication 6 dans laquelle la solution d'alcool polyvinylique aqueux contient un tensioactif non ionique selon un rapport pondéral de l'alcool polyvinylique au tensioactif non ionique d'au moins 5/1, ou contient un tensioactif cationique ou anionique selon un rapport pondéral de l'alcool polyvinylique au tensioactif cationique ou anionique d'au moins 2,5/1.

8. Un film multicouche pharmaceutiquement ou cosmétiquement actif comprenant un film multicouche comprenant une couche de silicone en contact fonctionnel avec une couche de poly(alcool vinylique), la couche de silicone comprenant une agrégation de la phase dispersée de la dispersion et la couche de poly(alcool vinylique) pouvant être **caractérisée par** un angle de contact avec l'eau (θ) de moins de 80 degrés (°) quand θ est mesuré conformément à ASTM D7334-08 30 secondes après qu'une gouttelette d'eau d'essai a été déposée dessus et contenant en outre un ingrédient pharmaceutiquement ou cosmétiquement actif, respectivement.

9. Le film multicouche pharmaceutiquement ou cosmétiquement actif de la revendication 8 dans lequel ladite couche de silicone est un matériau de silicone élastomérique formé par réaction de l'organopolysiloxane contenant de l'alcényle (a) et du siloxane contenant de la SiH (b).

10. Une méthode de masquage des rides cutanées chez un mammifère ayant besoin d'un tel traitement, la méthode comprenant l'application de manière topique d'une quantité efficace d'une dispersion sur la peau du mammifère dans laquelle la dispersion comprend une composition de silicone dispersée dans une phase aqueuse, la composition de silicone comprenant un produit d'une réaction (a) d'un organopolysiloxane contenant de l'alcényle ayant une moyenne par molécule d'au moins 2 groupes alcényle et (b) d'un siloxane contenant de la SiH ayant une moyenne par molécule d'au moins 2 groupes fonctionnels SiH, la composition de dispersion comprenant aussi un catalyseur d'hydrosilylation et de l'alcool polyvinylique, l'organopolysiloxane contenant de l'alcényle (a) et le siloxane contenant de la SiH (b) étant partiellement mis à réagir ensemble, et la composition de silicone étant stabilisée sous forme de dispersion par l'alcool polyvinylique dissous dans la phase aqueuse, dans laquelle la dispersion est exempte de tout tensioactif non polymérique.
